# EUROPEAN PATENT SPECIFICATION

(11) **EP 4 313 292 B1**
(45) Date of publication and mention of the grant of the patent: **18.02.2026**
(21) Application number: 22718185.6
(22) Date of filing: 25.03.2022
(51) Int. Cl.: A61P 1/00, A61P 9/00, A61P 11/00, A61P 27/00, C07D 401/14, C07D 403/06, C07D 403/14, A61K 31/506

(54) **INDOLINE DERIVATIVES AS DDR1 AND DDR2 INHIBITORS**
INDOLINDERIVATE ALS DDR1 UND DDR2 INHIBITOREN
DÉRIVÉS D'INDOLINE EN TANT QU'INHIBITEURS DDR1 ET DDR2

(30) Priority: 26.03.2021 EP 21165265
(43) Date of publication of application: 07.02.2024
(73) Proprietor: Chiesi Farmaceutici S.p.A., 43122 Parma (IT)
(72) Inventor: CARZANIGA, Laura, 43122 Parma (IT); RANCATI, Fabio, 43122 Parma (IT); RIZZI, Andrea, 43122 Parma (IT); KARAWAJCZYK, Anna, 43122 Parma (IT); WOLEK, Barbara Karolina, 43122 Parma (IT); MULLINS, Toby Mattew Grover, 43122 Parma (IT)
(74) Representative: Chiesi Farmaceutici S.p.A.
(86) International application number: PCT/EP2022/057938
(87) International publication number: WO 2022/200576

(56) References cited:
- DATABASE REGISTRY [online] CHEMICAL ABSTRACTS SERVICE, COLUMBUS, OHIO, US; 13 March 2008 (2008-03-13), XP002803789, Database accession no. 1007661-36-0
- DATABASE REGISTRY [online] CHEMICAL ABSTRACTS SERVICE, COLUMBUS, OHIO, US; 13 August 2003 (2003-08-13), XP002803790, Database accession no. 1007698-93-2
- DATABASE REGISTRY [online] CHEMICAL ABSTRACTS SERVICE, COLUMBUS, OHIO, US; 30 March 2008 (2008-03-30), XP002803791, Database accession no. 1010889-26-5
- DATABASE REGISTRY [online] CHEMICAL ABSTRACTS SERVICE, COLUMBUS, OHIO, US; 8 October 2008 (2008-10-08), XP002803792, Database accession no. 1058230-78-6
- DATABASE REGISTRY [online] CHEMICAL ABSTRACTS SERVICE, COLUMBUS, OHIO, US; 8 October 2008 (2008-10-08), XP002803793, Database accession no. 1058230-98-0
- DATABASE REGISTRY [online] CHEMICAL ABSTRACTS SERVICE, COLUMBUS, OHIO, US; 8 October 2008 (2008-10-08), XP002803794, Database accession no. 1058256-74-8
- DATABASE REGISTRY [online] CHEMICAL ABSTRACTS SERVICE, COLUMBUS, OHIO, US; 8 October 2008 (2008-10-08), XP002803795, Database accession no. 1058256-88-4
- DATABASE REGISTRY [online] CHEMICAL ABSTRACTS SERVICE, COLUMBUS, OHIO, US; 8 October 2008 (2008-10-08), XP002803796, Database accession no. 1058455-20-1
- DATABASE REGISTRY [online] CHEMICAL ABSTRACTS SERVICE, COLUMBUS, OHIO, US; 9 August 2013 (2013-08-09), XP002803797, Database accession no. 1448036-62-1
- DATABASE REGISTRY [online] CHEMICAL ABSTRACTS SERVICE, COLUMBUS, OHIO, US; 9 August 2013 (2013-08-09), XP002803798, Database accession no. 1448059-70-8
- ZHU DONGSHENG ET AL: "2-Amino-2,3-dihydro-1 H -indene-5-carboxamide-Based Discoidin Domain Receptor 1 (DDR1) Inhibitors: Design, Synthesis, and in Vivo Antipancreatic Cancer Efficacy", vol. 62, no. 16, 22 August 2019 (2019-08-22), pages 7431 - 7444, XP055828051, ISSN: 0022-2623, Retrieved from the Internet <URL:https://pubs.acs.org/doi/pdf/10.1021/acs.jmedchem.9b00365> DOI: 10.1021/acs.jmedchem.9b00365
- GUO JING ET AL: "A patent review of discoidin domain receptor 1 (DDR1) modulators (2014-present)", vol. 30, no. 5, 3 May 2020 (2020-05-03), GB, pages 341 - 350, XP055827937, ISSN: 1354-3776, Retrieved from the Internet <URL:https://www.tandfonline.com/doi/pdf/10.1080/13543776.2020.1732925?needAccess=true> DOI: 10.1080/13543776.2020.1732925

## Description

### FIELD OF THE INVENTION

The present invention relates to compounds inhibiting Discoidin Domain Receptors (DDR inhibitors), methods of preparing such compounds, pharmaceutical compositions containing them and therapeutic use thereof. The compounds of the invention may be useful for instance in the treatment of many disorders associated with DDR mechanisms.

### BACKGROUND OF THE INVENTION

Discoidin Domain Receptors (DDRs) are type I transmembrane receptor tyrosine kinase (RTKs). The DDR family comprises two distinct members, DDR1 and DDR2.

DDRs are unique receptors among the other members of the RTK superfamily, in that DDRs are activated by collagen whereas other members of the RTK superfamily are typically activated by soluble peptide-like growth factors (see Vogel, W. (1997) Mol. Cell 1, 13-23; Shrivastava A. Mol Cell. 1997; 1:25-34.). Moreover, DDRs are unusual RTKs also because they form ligand-independent stable dimers that are non-covalently linked (see Noordeen, N. A.(2006) J. Biol. Chem. 281, 22744-22751; Mihai C. J Mol Biol. 2009; 385:432-445).

The DDR1 subfamily is composed of five membrane-anchored isoforms, and the DDR2 subfamily is represented by a single protein. The five DDR1 isoforms all have in common the extracellular and transmembrane domains but differ in the cytoplasmic region (see Valiathan, R. R. (2012) Cancer Metastasis Rev. 31, 295-321; Alves, F. (2001) FASEB J. 15, 1321-1323).

DDR receptor family has been found involved in a series of fibrotic diseases, such as pulmonary fibrosis, and in particular idiopathic pulmonary fibrosis (IPF). The first evidence for a protective role of DDR1 deletion in lung fibrosis was generated in 2006 by the research group of Dr. Vogel (see Avivi-Green C, Am J Respir Crit Care Med 2006;174:420-427). The authors demonstrated that DDR1-null mice were largely protected against bleomycin (BLM)-induced injury. Furthermore, myofibroblast expansion and apoptosis were much lower in these animals compared with their wild-type counterparts. Absence of inflammation in knockout mice was confirmed by lavage cell count and cytokines ELISA. These results indicated that DDR1 expression is a prerequisite for the development of lung inflammation and fibrosis.

DDR2 deficiency or downregulation reduces bleomycin-induced lung fibrosis (see Zhao H, Bian H, Bu X, Zhang S, Zhang P, Yu J, et al Mol Ther 2016; 24:1734-1744). Zhao et al, demonstrated that DDR2 plays a critical role in the induction of fibrosis and angiogenesis in the lung, in particular that DDR2 synergizes with transforming growth factor (TGF)-β to induce myofibroblast differentiation. Furthermore, they showed that treatment of injured mice with specific siRNA against DDR2 exhibited therapeutic efficacy against lung fibrosis. In a second publication, Jia et al showed that mice lacking DDR2 are protected from bleomycin-induced lung fibrosis (see Jia S, Am J Respir Cell Mol Biol 2018;59:295-305). In addition, DDR2-null fibroblasts are significantly more prone to apoptosis than wild-type fibroblasts, supporting a paradigm in which fibroblast resistance to apoptosis is critical for progression of fibrosis.

Some compounds have been described in the literature as DDR1 or DDR2 antagonists.

WO2016064970 (Guangzhou) discloses tetrahydroisoquinoline-7-carboxamides as selective DDR1 inhibitors useful as therapeutic agents for preventing and treating inflammation, liver fibrosis, kidney fibrosis, lung fibrosis, skin scar, atherosclerosis and cancer.

From the same inventors of Guangzhou, Wang Z. et al. in J. Med. Chem. 2016, 59, 5911-5916 discloses tetrahydroisoquinoline-7-carboxamides as selective DDR1 inhibitors. Zhu et al., J. Med. Chem. 2019, 62, 7431-7444 discloses indene derivatives as DDR1 inhibitors.

Of note, antagonizing the DDR receptors may be useful for the treatment of fibrosis and disease, disorder and conditions that result from fibrosis and even more antagonizing both receptors DDR1 and DDR2 may be particularly efficacious in the treatment of the above-mentioned disease, disorder and conditions.

Several efforts have been done in the past years to develop novel DDR1 and DDR2 receptor antagonists useful for the treatment of several disease and some of those compounds have shown efficacy also in humans.

Despite the above cited prior art, there remains a potential for developing selective inhibitors of both receptors DDR1 and DDR2 useful for the treatment of diseases or conditions associated with a dysregulation of DDR receptors, in the respiratory field, in particular idiopathic pulmonary fibrosis (IPF), to be administered by the inhalation route and characterized by a good inhalatory profile, that corresponds to a good activity in the lung, a good lung retention and to a low metabolic stability in order to minimize the systemic exposure and correlated safety issues.

In this direction, we have surprisingly found a new series of compounds of general formula (I), as herein below reported, that solves the problem of providing inhibitors for receptors DDR1 and DDR2 for administration by inhalation, that are active as selective inhibitors of DDR1 and DDR2 receptors with respect to other human protein kinases. Such compounds show high potency, good inhalatory profile, low metabolic stability, low systemic exposure, improved safety and tolerability.

### SUMMARY OF THE INVENTION

In a first aspect the present invention relates to a compound of formula (I) 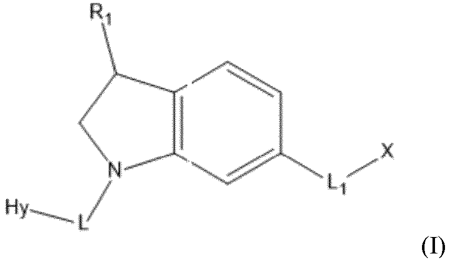 wherein
**R₁** is H or -(C₁-C₄)alkyl;
**L** -CH₂;
**L₁** is selected from the group consisting of -C(O)NH- and -NHC(O)-;
**Hy** is a monocyclic heteroaryl optionally substituted by one or more groups selected from -(CH₂)ₙNR₄R₅, -C(O)NR₄R₅, -(C₁-C₄)alkyl, -NR₄(CO)R₅, -(C₁-C₄)alkylene-O-(C₁-C₄)alkyl and -(C₄-C₇)heterocycloalkyl optionally substituted by one or more groups selected from -(C₁-C₄)alkyl and oxo;
**n** is 0, 1 or 2;
**R₄** is H or -(C₁-C₄)alkyl;
**R₅** is H or is selected from the group consisting of (C₃-C₇)cycloalkyl, -(C₁-C₄)alkyl and heteroaryl optionally substituted by one or more groups selected from -(C₁-C₄)alkyl, -(C₁-C₄)alkylene-NR₁R₄ and -(C₁-C₄)alkylene-O-(C₁-C₄)alkyl;
X is 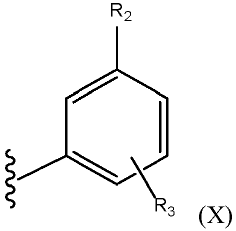 wherein **R₂** is selected from the group consisting of -(C₁-C₄)alkyl, -O(C₁-C₄)haloalkyl, halogen atoms, -(C₁-C₄)haloalkyl, -(C₃-C₇)cycloalkyl and -O(C₃-C₇)cycloalkyl;
**R₃** is H or is selected from the group consisting of -O(C₁-C₄)alkyl, heteroaryl optionally substituted by one or more -(C₁-C₄)alkyl, and (C₄-C₇)heterocycloalkyl-(C₁-C₄)alkylene-, wherein said -(C₄-C₇)heterocycloalkyl is optionally substituted by one or more groups selected from -(C₁-C₄)alkyl and -O(C₁-C₄)alkyl;
and pharmaceutically acceptable salts thereof.

In a second aspect, the invention refers to a pharmaceutical composition comprising a compound of formula (I) and pharmaceutically acceptable salts thereof in a mixture with one or more pharmaceutically acceptable carrier or excipient.

In a third aspect, the invention refers to a compound of formula (I) and pharmaceutically acceptable salts or to a pharmaceutical composition comprising a compound of formula (I) and pharmaceutically acceptable salts thereof for use as a medicament.

In a further aspect, the invention refers to a compound of formula (I) and pharmaceutically acceptable salts thereof or to a pharmaceutical composition comprising a compound of formula (I) and pharmaceutically acceptable salts thereof for use in preventing and/or treating a disease, disorder or condition associated with dysregulation of DDR.

In a further aspect, the invention refers to a compound of formula (I) and pharmaceutically acceptable salts thereof or to a pharmaceutical composition comprising a compound of formula (I) and pharmaceutically acceptable salts thereof for use in the prevention and/or treatment of fibrosis and/or diseases, disorders, or conditions that involve fibrosis.

In a further aspect, the invention refers to a compound of formula (I) and pharmaceutically acceptable salts thereof or to a pharmaceutical composition comprising a compound of formula (I) and pharmaceutically acceptable salts thereof for use in the prevention and/or treatment idiopathic pulmonary fibrosis (IPF).

### DETAILED DESCRIPTION OF THE INVENTION

### Definitions

Unless otherwise specified, the compound of formula (I) of the present invention is intended to include also its stereoisomers or pharmaceutically acceptable salts thereof.

Unless otherwise specified, the compound of formula (I) of the present invention is intended to include also the compounds of formula (Ia), (Ia1), (Ia1'), (Ia1"), (Ib), (Ib1), (Ib1').

The term "pharmaceutically acceptable salts", as used herein, refers to derivatives of compounds of formula (I) wherein the parent compound is suitably modified by converting any of the free acid or basic group, if present, into the corresponding addition salt with any base or acid conventionally intended as being pharmaceutically acceptable.

Suitable examples of said salts may thus include mineral or organic acid addition salts of basic residues such as amino groups, as well as mineral or organic basic addition salts of acid residues such as carboxylic groups.

Cations of inorganic bases which can be suitably used to prepare salts comprise ions of alkali or alkaline earth metals such as potassium, sodium, calcium or magnesium.

Those obtained by reacting the main compound, functioning as a base, with an inorganic or organic acid to form a salt comprise, for example, salts of hydrochloric acid, hydrobromic acid, sulfuric acid, phosphoric acid, methane sulfonic acid, camphor sulfonic acid, acetic acid, oxalic acid, maleic acid, fumaric acid, succinic acid and citric acid.

The term "stereoisomer" refers to isomers of identical constitution that differ in the arrangement of their atoms in space. Enantiomers and diastereomers are examples of stereoisomers.

The term "enantiomer" refers to one of a pair of molecular species that are mirror images of each other and are not superimposable.

The term "diastereomer" refers to stereoisomers that are not mirror images.

The term "racemate" or "racemic mixture" refers to a composition composed of equimolar quantities of two enantiomeric species, wherein the composition is devoid of optical activity.

The term "halogen" or "halogen atoms" or "halo" as used herein includes fluorine, chlorine, bromine, and iodine atom.

The term "(Cₓ-C_{y})alkyl" wherein x and y are integers, refers to a straight or branched chain alkyl group having from x to y carbon atoms. Thus, when x is 1 and y is 4, for example, the term comprises methyl, ethyl, n-propyl, isopropyl, n-butyl, isobutyl, sec-butyl, and t-butyl.

The term "O(Cₓ-C_{y})alkyl" wherein x and y are integers, refers to the above defined "(Cₓ-C_{y})alkyl" groups wherein a carbon atom is linked to an oxygen atom, e.g. ethoxy.

The term "(Cₓ-C_{y})alkylene" wherein x and y are integers, refers to a (Cₓ-C_{y})alkyl radical having in total two unsatisfied valencies.

The term "(Cₓ-C_{y})haloalkyl" wherein x and y are integers, refers to the above defined "(Cₓ-C_{y}) alkyl" groups wherein one or more hydrogen atoms are replaced by one or more halogen atoms, which can be the same or different. Examples of said "(Cₓ-C_{y})haloalkyl" groups may thus include halogenated, poly-halogenated and fully halogenated alkyl groups wherein all hydrogen atoms are replaced by halogen atoms, e.g. trifluoromethyl.

The term "O(Cₓ-C_{y})haloalkyl" wherein x and y are integers, refers to the above defined "(Cₓ-C_{y})haloalkyl" groups wherein the carbon atom is linked to an oxygen atom.

Examples of said "O(Cₓ-C_{y})haloalkyl" groups may thus include halogenated, poly-halogenated and fully halogenated alkyl groups wherein all hydrogen atoms are replaced by halogen atoms, e.g. trifluoromethoxy.

The term "(Cₓ-C_{y})cycloalkyl" wherein x and y are integers, refers to saturated cyclic hydrocarbon groups containing the indicated number of carbon atoms in the ring. Examples include e.g. cyclopropyl, cyclobutyl, cyclopentyl, cyclohexyl, cycloheptyl.

The term "O(Cₓ-C_{y})cycloalkyl" wherein x and y are integers, refers to the above defined "(Cₓ-C_{y})cycloalkyl" groups, wherein the carbon atom is linked to an oxygen atom. Examples include, e.g. cyclopropyloxy.

The term "aryl" refers to mono cyclic carbon ring systems which have 6 ring atoms wherein the ring is aromatic. Examples of suitable aryl monocyclic ring systems include, for instance, phenyl.

The term "heteroaryl" refers to a mono- or bi-cyclic aromatic group containing one or more heteroatoms selected from S, N and O, and includes groups having two such monocyclic rings, or one such monocyclic ring and one monocyclic aryl ring, which are fused through a common bond. Examples include, e.g. imidazolyl, pyrimidinyl, pyrazolyl, aminopyrazinyl, pyridyl.

The term "-(Cₓ-C_{y})heterocycloalkyl" wherein x and y are integers, refers to saturated or partially unsaturated monocyclic (Cₓ-C_{y})cycloalkyl groups in which at least one ring carbon atom is replaced by at least one heteroatom (e.g. N, S or O) or may bear an -oxo (=O) substituent group. Said heterocycloalkyl may be further optionally substituted on the available positions in the ring, namely on a carbon atom, or on a heteroatom available for substitution. Substitution on a carbon atom includes spiro disubstitution as well as substitution on two adjacent carbon atoms, in both cases thus form additional condensed 5 to 6 membered heterocyclic ring. Examples include, e.g. piperazinyl and oxopiperazinyl.

The term "(Cₓ-C_{y})heterocycloalkyl-(Cₓ-C_{y})alkylene-" refers to an heterocycloalkyl linked to a straight-chained or branched (Cₓ-C_{y})alkylene group having from x to y carbon atoms. Examples include, e.g. (4-methylpiperazin-1-yl)methyl.

A bond pointing to a wavy or squiggly line, such as 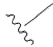 as used in structural formulas herein, depicts the bond that is the point of attachment of the moiety or substituent to the core or backbone structure.

When referring to substituents, a dash ("-") that is not between two letters, words, or symbols is meant to represent the point of attachment for such substituents.

The carbonyl group is herein preferably represented as -C(O)- as an alternative to the other common representations such as -CO-, -(CO)- or -C(=O)-.

When referring to the group -C(O)NH- or -NHC(O)- of **L₁** of formula (I), the left dash ("-") of the group is the bond between the group and the indoline core, and the right dash ("-") of the group is the bond between the group and the **X** group. Accordingly, when referring to -C(O)NH- of **L₁** of formula **(I),** the carbon is bonded to the indoline core, and the nitrogen is bonded to the **X** group. When referring to -NHC(O)- of **L₁** of formula (I), the nitrogen is bonded to the indoline core, and the carbon is bonded to the **X** group.

Whenever basic amino groups are present in the compounds of formula (I), physiologically acceptable anions may be present, selected among chloride, bromide, iodide, trifluoroacetate, formate, sulfate, phosphate, methanesulfonate, nitrate, maleate, acetate, citrate, fumarate, tartrate, oxalate, succinate, benzoate, p-toluenesulfonate, pamoate and naphthalene disulfonate. Likewise, in the presence of acidic groups, corresponding physiological cation salts may be present as well, for instance including alkaline or alkaline earth metal ions.

The term "half maximal inhibitory concentration" (IC50) indicates the concentration of a particular compound or molecule required for obtaining 50% inhibition of a biological process in vitro.

The term "Ki" indicates the dissociation constant for the enzyme-inhibitor complex, expressed in molar units. It is an indicator of the binding affinity between inhibitor and DDR1 or DDR2 receptors.

The present invention refers to novel compounds differing from the structures disclosed in the art at least for a common new core scaffold. In fact the invention relates to compounds that are indoline derivatives, wherein the nitrogen of the indoline is linked to a monocyclic heteroaryl ring Hy through a linker -CH₂- or -C(O)- represented by the general formula (I) as herein below described in details, which are endowed with an inhhibitory activity on receptors DDR1 and DDR2.

The compounds of the invention are active as selective inhibitors of DDR1 and DDR2 receptors with respect to other human protein kinases, they are potent and show good inhalatory profile, low metabolic stability, low systemic exposure, improved safety and tolerability.

In this respect, the state of the art does not describe or suggests indoline derivatives of general formula (I) of the present invention having inhibitory activity on receptors DDR1 and DDR2 which represent a solution to the aforementioned need.

Guangzhou and Wang disclose tetrahydroisoquinoline-7-carboxamides as selective DDR1 Inhibitors.

Of note, in the compounds of formula (I) according to present invention, the presence of at least the indoline moiety and a spacer -CH₂- between the nitrogen atom of the pyrrolidine and the heteroaryl group Hy unexpectedly and remarkably determines high affinities for both DDR1 and DDR2 receptors and relevant inhibitory activity on the DDR1 and, in addition, also on the DDR2 receptors in the cellular assay.

Indeed, as detailed in the experimental part below, the compounds of formula (I) of the present invention are able to act as antagonist of both DDR1 and DDR2 receptors in a substantive and effective way. In particular, Table 5 below shows that for the compounds of the present invention, both the affinity for either DDR1 and DDR2 receptors and the inhibitory activity on either DDR1 and DDR2 receptors are below about 80 nM respectively in the binding (expressed as Ki) and the cell based assays (expressed as IC50). This confirms that the compounds of formula (I) are able to inhibit the two isoforms of DDR receptor mainly involved in fibrosis and fibrosis-related diseases. Accordingly, the compounds of formula (I) can be used in the treatment of fibrosis, in particular pulmonary fibrosis, when DDR1 and DDR2 are involved.

Moreover, as indicated in the experimental part, in particular comparative examples in Table 6, it is shown that conversely to the compounds of Examples C1 to C3 characterized by lacking a linker between the indoline ring and Hy group, the presence of a -CH₂- linker in that position in the present invention compounds surprisingly and remarkably determines a relevant increase in the inhibitory activity against the DDR1 and DDR2.

Advantageously, the compounds of the present invention are endowed with a very high affinity and potency for the DDR1 and DDR2 as shown respectively in the binding and cell based assays, they could be administered in human at a lower dosage respect to the compounds of the prior art, thus reducing the adverse events that might occur administering higher dosages of drug.

In addition to their high affinity for the DDR1 and DDR2 receptors and being notably potent in exerting their inhibitory activity against both DDR1 and DDR2 receptors, the compounds of the present invention are also characterized by a good inhalatory profile, that permits to act effectively on the lung compartment and have, at the same time, a low metabolic stability, that allows to minimize the drawbacks associated with the systemic exposure, such as safety and tolerability issues.

Therefore, the compounds of the present invention are particularly appreciated by the skilled person when looking at a suitable and efficacious compounds useful for the treatment of fibrosis, in particular idiopatic pulmonary fibrosis, administered by the inhalation route and characterized by a good inhalatory profile, that corresponds to a good activity on the lung, a good lung retention and to a low metabolic stability, that minimizes the systemic exposure and correlated safety issues.

Thus, in one aspect the present invention relates to a compound of general formula (I) 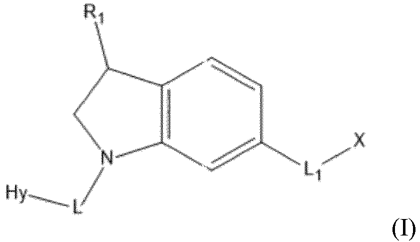 wherein
**R₁** is H or -(C₁-C₄)alkyl;
**L** is CH₂ ;
**L₁** is selected from the group consisting of -C(O)NH- and -NHC(O)-;
**Hy** is a monocyclic heteroaryl optionally substituted by one or more groups selected from -(CH₂)ₙNR₄R₅, -C(O)NR₄R₅, -(C₁-C₄)alkyl, -NR₄(CO)R₅, -(C₁-C₄)alkylene-O-(C₁-C₄)alkyl and -(C₄-C₇)heterocycloalkyl optionally substituted by one or more groups selected from -(C₁-C₄)alkyl and oxo;
**n** is 0, 1 or 2;
**R₄** is H or -(C₁-C₄)alkyl;
**R₅** is H or is selected from the group consisting of (C₃-C₇)cycloalkyl, -(C₁-C₄)alkyl and heteroaryl optionally substituted by one or more groups selected from -(C₁-C₄)alkyl, - (C₁-C₄)alkylene-NR₁R₄ and -(C₁-C₄)alkylene-O-(C₁-C₄)alkyl;
**X** is 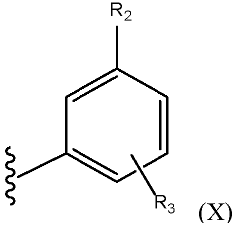 wherein **R₂** is selected from the group consisting of -(C₁-C₄)alkyl, -O(C₁-C₄)haloalkyl, halogen atoms, -(C₁-C₄)haloalkyl, -(C₃-C₇)cycloalkyl and -O(C₃-C₇)cycloalkyl;
**R₃** is H or is selected from the group consisting of -O(C₁-C₄)alkyl, heteroaryl optionally substituted by one or more -(C₁-C₄)alkyl, and (C₄-C₇)heterocycloalkyl-(C₁-C₄)alkylene-, wherein said -(C₄-C₇)heterocycloalkyl is optionally substituted by one or more groups selected from -(C₁-C₄)alkyl and -O(C₁-C₄)alkyl;
and pharmaceutically acceptable salts thereof.

In one preferred embodiment **R₁** is H.

In another preferred embodiment **Hy** is a monocyclic heteroaryl wherein the heteroatom is represented by one or more N atoms.

In another preferred embodiment, the invention refers to a compound of general formula (I) wherein **Hy** is selected from the group consisting of 3-aminopyrazin-2-yl, pyrimidin-5-yl, 2-(methylcarbamoyl)pyridin-4-yl, -N-methyl-4-picolinamide, 4-methyl-3-oxopiperazin-1-yl)pyridin-3-yl, 2-aminopyrimidin-5-yl, 6-aminopyridin-3-yl, 4-aminopyrimidin-5-yl, 5-aminopyrazin-2-yl, 2-(methylamino)pyrimidin-5-yl, 6-acetamidopyridin-3-yl, 6-(methylamino)pyridin-3-yl, 2-acetamidopyrimidin-5-yl, 2-((1-(2-methoxyethyl)-1H-pyrazol-4-yl)amino)pyrimidin-5-yl, 5-carbamoylpyridin-3-yl, 2-((1-(2-(dimethylamino)ethyl)-1H-pyrazol-4-yl)amino)pyrimidin-5-yl and (2-((1-methyl-1H-pyrazol-4-yl)amino)pyrimidin-5-yl.

In a further preferred embodiment, the invention refers to a compound of general formula (I) wherein **X** is selected from the group consisting of ((4-methylpiperazin-1-yl)methyl)-5-(trifluoromethyl)phen-3-yl, 3-(trifluoromethyl)phenyl, 4-((4-methylpiperazin-1-yl)methyl)-3-(trifluoromethyl)phenyl and (4-methyl-1H-imidazol-1-yl)-5-(trifluoromethyl)phen-3-yl.

According to a preferred embodiment, the invention refers to at least one of the compounds listed in the Table 1 below. These compounds are active on receptors DDR1 and DDR2, as shown in Table 5.

**Table 1: List of compounds of formula (I)**

| **Ex. No.** | **Structure** | **Chemical Name** |
|---|---|---|
| 1 | 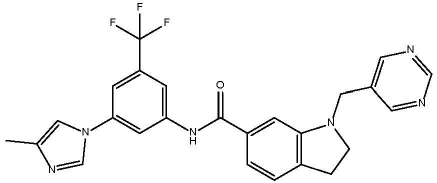 | N-(3-(4-methyl-1H-imidazol-1-yl)-5-(trifluoromethyl)phenyl)-1-(pyrimidin-5-ylmethyl)indoline-6-carboxamide |
| 2 | 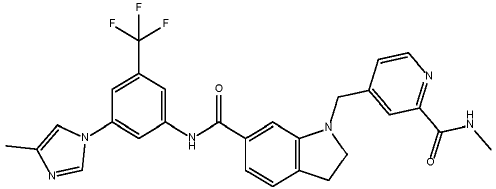 | N-(3-(4-methyl-1H-imidazol-1-yl)-5-(trifluoromethyl)phenyl)-1-((2-(methylcarbamoyl)pyridi n-4-yl)methyl)indoline-6-carboxamide |
| 3 | 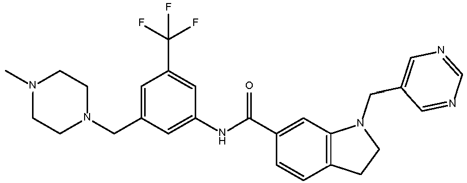 | N-(3-((4-methylpiperazin-1-yl)methyl)-5-(trifluoromethyl)phenyl)-1-(pyrimidin-5-ylmethyl)indoline-6-carboxamide |
| 4 | 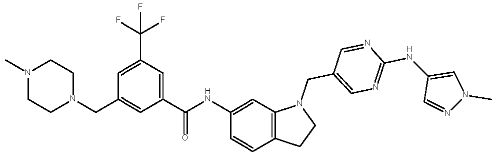 | N-(1-((2-((1-methyl-1H-pyrazol-4-yl)amino)pyrimidin-5-yl)methyl)indolin-6-yl)-3-((4-methylpiperazin-1-yl)methyl)-5-(trifluoromethyl)benzami de |
| 5 | 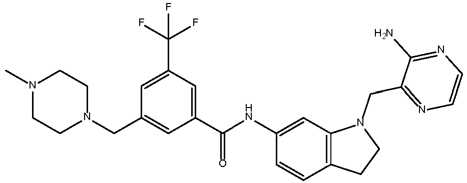 | N-(1-((3-aminopyrazin-2-yl)methyl)indolin-6-yl)-3-((4-methylpiperazin-1-yl)methyl)-5-(trifluoromethyl)benzami de |
| 6 | 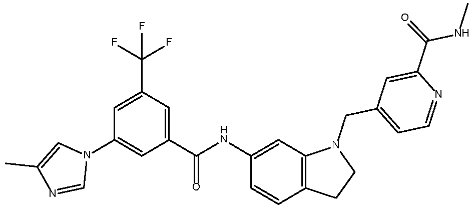 | N-methyl-4-((6-(3-(4-methyl-1H-imidazol-1-yl)-5-(trifluoromethyl)benzami do)indolin-1-yl)methyl)picolinamide |
| 7 | 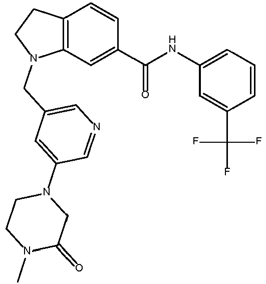 | 1-((5-(4-methyl-3-oxopiperazin-1-yl)pyridin-3-yl)methyl)-N-(3-(trifluoromethyl)phenyl)i ndoline-6-carboxamide |
| 8 | 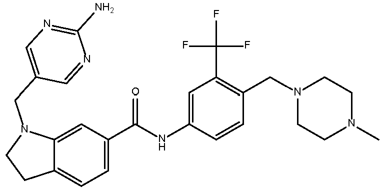 | 1-((2-aminopyrimidin-5-yl)methyl)-N-(4-((4-methylpiperazin-1-yl)methyl)-3-(trifluoromethyl)phenyl)i ndoline-6-carboxamide |
| 9 | 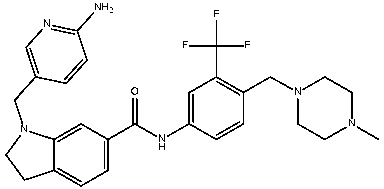 | 1-((6-aminopyridin-3-yl)methyl)-N-(4-((4-methylpiperazin-1-yl)methyl)-3-(trifluoromethyl)phenyl)i ndoline-6-carboxamide |
| 10 | 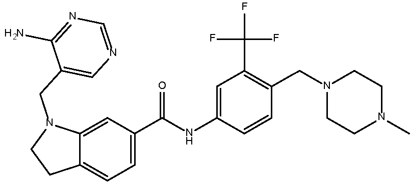 | 1-((4-aminopyrimidin-5-yl)methyl)-N-(4-((4-methylpiperazin-1-yl)methyl)-3-(trifluoromethyl)phenyl)i ndoline-6-carboxamide |
| 11 | 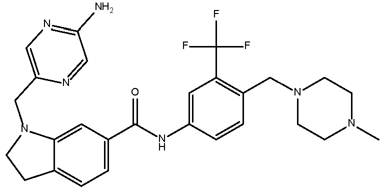 | 1-((5-aminopyrazin-2-yl)methyl)-N-(4-((4-methylpiperazin-1-yl)methyl)-3-(trifluoromethyl)phenyl)i ndoline-6-carboxamide |
| 12 | 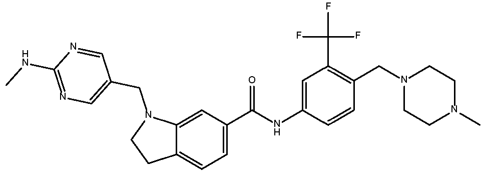 | 1-((2-(methylamino)pyrimidin-5-yl)methyl)-N-(4-((4-methylpiperazin-1-yl)methyl)-3-(trifluoromethyl)phenyl)i ndoline-6-carboxamide |
| 13 | 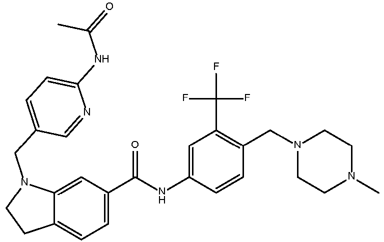 | 1-((6-acetamidopyridin-3-yl)methyl)-N-(4-((4-methylpiperazin-1-yl)methyl)-3-(trifluoromethyl)phenyl)i ndoline-6-carboxamide |
| 14 | 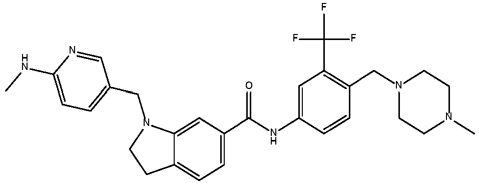 | 1-((6-(methylamino)pyridin-3-yl)methyl)-N-(4-((4-methylpiperazin-1-yl)methyl)-3-(trifluoromethyl)phenyl)i ndoline-6-carboxamide |
| 15 | 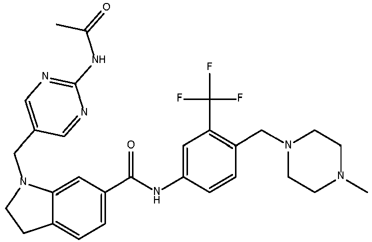 | 1-((2-acetamidopyrimidin-5-yl)methyl)-N-(4-((4-methylpiperazin-1-yl)methyl)-3-(trifluoromethyl)phenyl)i ndoline-6-carboxamide |
| 16 | 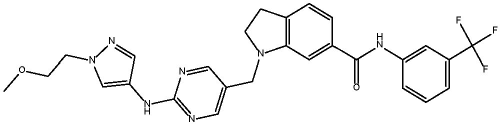 | 1-((2-((1-(2-methoxyethyl)-1H-pyrazol-4-yl)amino)pyrimidin-5-yl)methyl)-N-(3-(trifluoromethyl)phenyl)i ndoline-6-carboxamide |
| 17 | 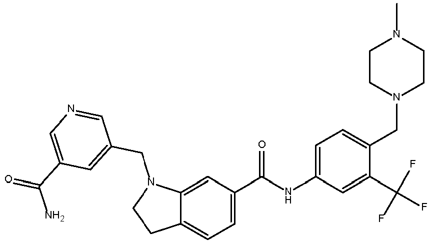 | 1-((5-carbamoylpyridin-3-yl)methyl)-N-(4-((4-methylpiperazin-1-yl)methyl)-3-(trifluoromethyl)phenyl)i ndoline-6-carboxamide |
| 18 | 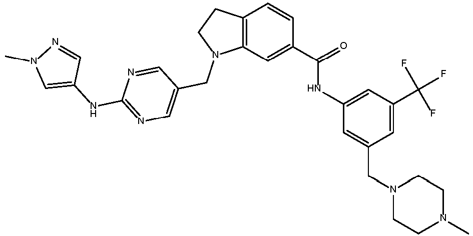 | 1-((2-((1-methyl-1H-pyrazol-4-yl)amino)pyrimidin-5-yl)methyl)-N-(3-((4-methylpiperazin-1-yl)methyl)-5-(trifluoromethyl)phenyl)i ndoline-6-carboxamide |
| 19 | 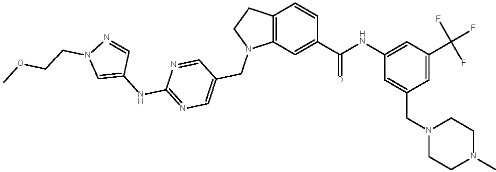 | 1-((2-((1-(2-methoxyethyl)-1H-pyrazol-4-yl)amino)pyrimidin-5-yl)methyl)-N-(3-((4-methylpiperazin-1-yl)methyl)-5-(trifluoromethyl)phenyl)i ndoline-6-carboxamide |
| 20 | 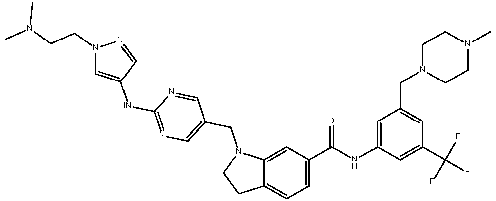 | 1-((2-((1-(2-(dimethylamino)ethyl)-1H-pyrazol-4-yl)amino)pyrimidin-5-yl)methyl)-N-(3-((4-methylpiperazin-1-yl)methyl)-5-(trifluoromethyl)phenyl)ind oline-6-carboxamide |
| 21 | 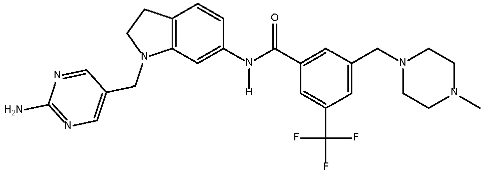 | N-(1-((2-aminopyrimidin-5-yl)methyl)indolin-6-yl)-3-((4-methylpiperazin-1-yl)methyl)-5-(trifluoromethyl)benzami de |

In a further preferred embodiment, the present invention relates to a compound of general formula (I) wherein **L₁** is -C(O)NH-, **L** is -CH₂-, represented by the formula (Ia) 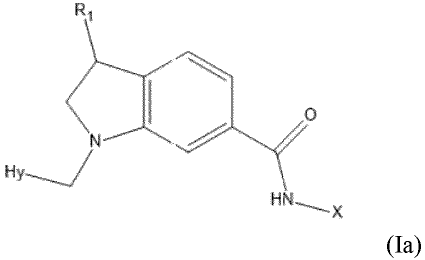 wherein **Hy**, **R₁** and X are as defined above.

In another particularly preferred embodiment the present invention refers to a compound of formula (Ia), wherein X is 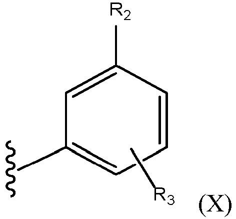 represented by the formula (Ia1) 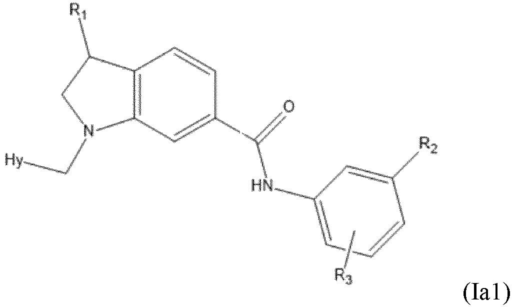 wherein **Hy, R₁, R₂** and **R₃** are as defined above.

In another preferred embodiment the present invention refers to a compound of formula (I), wherein **L₁** is -C(O)NH-, **L** is -CH₂-, X is X' 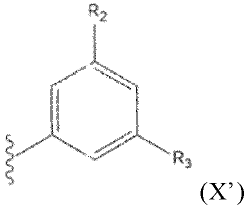 represented the by formula (Ia1') 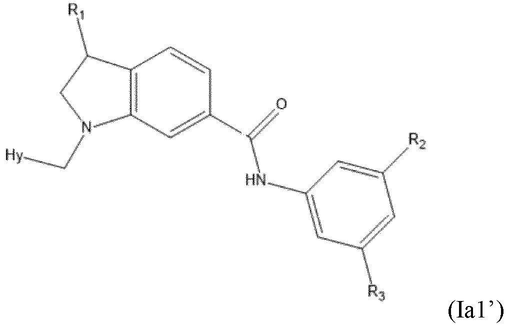 wherein **Hy, R₁, R₂** and **R₃** are as defined above.

In another particularly preferred embodiment the present invention refers to a compound of formula (Ia1') wherein **R₂** is -CF₃ and **R₃** is selected from the group consisting of heteroaryl optionally substituted by one or more -(C₁-C₄)alkyl, and (C₄-C₇)heterocycloalkyl-(C₁-C₄)alkylene-, wherein said -(C₄-C₇)heterocycloalkyl is optionally substituted by one or more -(C₁-C₄)alkyl, and pharmaceutically acceptable salts thereof.

In an even more particularly preferred embodiment the present invention refers to a compound of formula (Ia1') wherein **R₂** is -CF₃ and **R₃** is selected from the group consisting of 4-methyl-1H-imidazol-1-yl and (4-methylpiperazin-1-yl)methyl.

In a further preferred embodiment, the invention refers to a compound of general formula (Ia1') wherein **Hy** is selected from the group consisting of 2-(methylcarbamoyl)pyridin-4-yl, 2-((1-(2-methoxyethyl)-1H-pyrazol-4-yl)amino)pyrimidin-5-yl, 2-((1-methyl-1H-pyrazol-4-yl)amino)pyrimidin-5-yl, 2-((1-(2-(dimethylamino)ethyl)-1H-pyrazol-4-yl)amino)pyrimidin-5-yl, 2-aminopyrimidin-5-yl and pyrimidin-5-yl-methyl.

According to a preferred embodiment, the invention refers to at least one of the compounds of formula (Ia1') listed in the Table 2 below and pharmaceutically acceptable salts thereof. These compounds are active on receptors DDR1 and DDR2, as shown in Table 5.

**Table 2: List of compounds of formula (Ia1')**

| **Ex. No.** | **Structure** | **Chemical Name** |
|---|---|---|
| 1 | 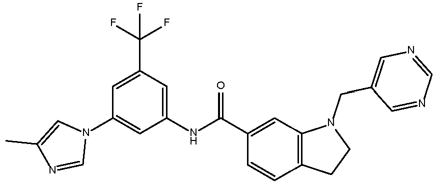 | N-(3-(4-methyl-1H-imidazol-1-yl)-5-(trifluoromethyl)phe nyl)-1-(pyrimidin-5-ylmethyl)indoline-6-carboxamide |
| 2 | 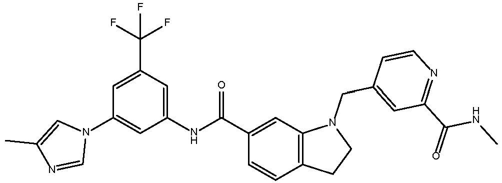 | N-(3-(4-methyl-1H-imidazol-1-yl)-5-(trifluoromethyl)phe nyl)-1-((2-(methylcarbamoyl)p yridin-4-yl)methyl)indoline-6-carboxamide |
| 3 | 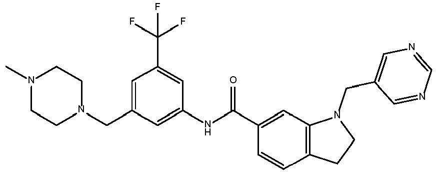 | N-(3-((4-methylpiperazin-1-yl)methyl)-5-(trifluoromethyl)phenyl )-1-(pyrimidin-5-ylmethyl)indoline-6-carboxamide |
| 18 | 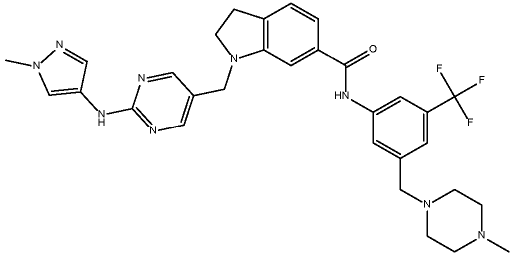 | 1-((2-((1-methyl-1H-pyrazol-4-yl)amino)pyrimidin-5-yl)methyl)-N-(3-((4-methylpiperazin-1-yl)methyl)-5-(trifluoromethyl)phenyl )indoline-6-carboxamide |
| 19 | 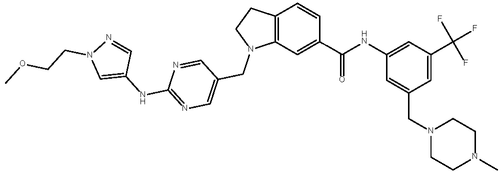 | 1-((2-((1-(2-methoxyethyl)-1H-pyrazol-4-yl)amino)pyrimidin-5-yl)methyl)-N-(3-((4-methylpiperazin-1-yl)methyl)-5-(trifluoromethyl)phenyl )indoline-6-carboxamide |
| 20 | 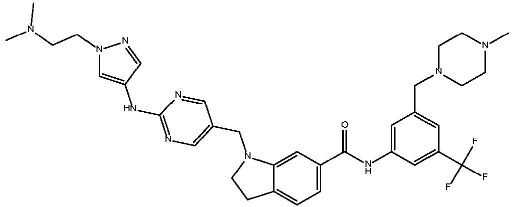 | 1-((2-((1-(2-(dimethylamino)ethyl)-1H-pyrazol-4-yl)amino)pyrimidin-5-yl)methyl)-N-(3-((4-methylpiperazin-1-yl)methyl)-5-(trifluoromethyl)phenyl )indoline-6-carboxamide |

In an another preferred embodiment the present invention refers to a compound of formula (Ia), wherein X is X'' 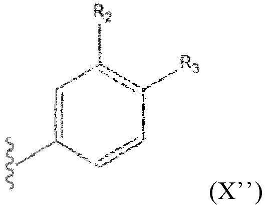 represented by the formula (Ia1") 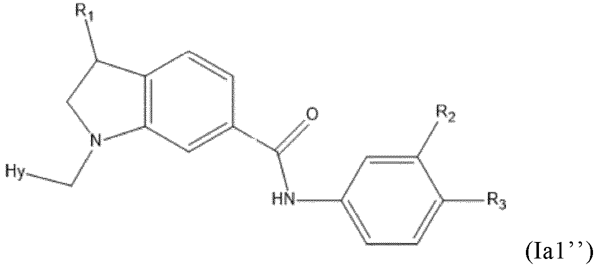 wherein **Hy, R₁, R₂** and **R₃** are as defined above.

In another particularly preferred embodiment the present invention refers to a compound of formula (Ia1") wherein **Hy** is selected from the group consisting of 2-aminopyrimidin-5-yl, 6-aminopyridin-3-yl, 4-aminopyrimidin-5-yl, 2-(methylamino)pyrimidin-5-yl, 6-acetamidopyridin-3-yl, 6-(methylamino)pyridin-3-yl, 2-acetamidopyrimidin-5-yl, and 5-aminopyrazin-2-yl, **R₂** is trifluoromethyl and **R₃** is (4-methylpiperazin-1-yl)methyl.

According to a preferred embodiment, the invention refers to at least one of the compounds of formula (Ia1") listed in the Table 7 below and pharmaceutically acceptable salts thereof. These compounds are active on receptors DDR1 and DDR2, as shown in Table 5.

**Table 7: List of compounds of formula (Ia1")**

| **Ex. No.** | **Structure** | **Chemical Name** |
|---|---|---|
| 8 | 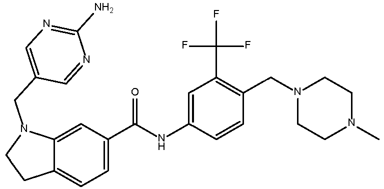 | 1-((2-aminopyrimidin-5-yl)methyl)-N-(4-((4-methylpiperazin-1-yl)methyl)-3-(trifluoromethyl)phe nyl)indoline-6-carboxamide |
| 9 | 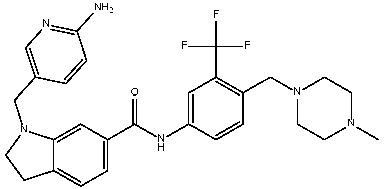 | 1-((6-aminopyridin-3-yl)methyl)-N-(4-((4-methylpiperazin-1-yl)methyl)-3-(trifluoromethyl)phe nyl)indoline-6-carboxamide |
| 10 | 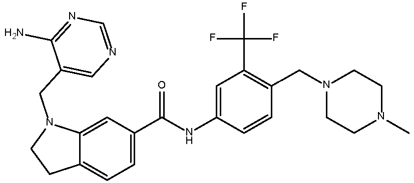 | 1-((4-aminopyrimidin-5-yl)methyl)-N-(4-((4-methylpiperazin-1-yl)methyl)-3-(trifluoromethyl)phe nyl)indoline-6-carboxamide |
| 11 | 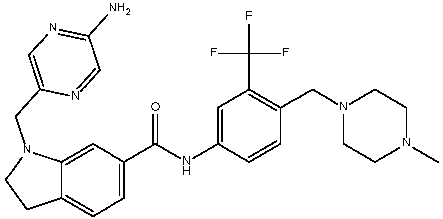 | 1-((5-aminopyrazin-2-yl)methyl)-N-(4-((4-methylpiperazin-1-yl)methyl)-3-(trifluoromethyl)phenyl )indoline-6-carboxamide |
| 12 | 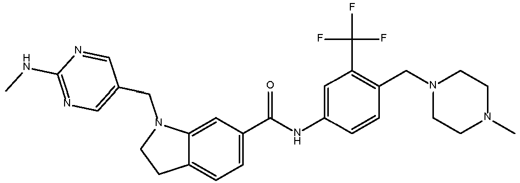 | 1-((2-(methylamino)pyrim idin-5-yl)methyl)-N-(4-((4-methylpiperazin-1-yl)methyl)-3-(trifluoromethyl)phe nyl)indoline-6-carboxamide |
| 13 | 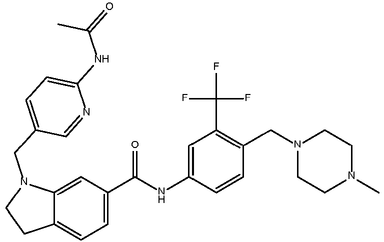 | 1-((6-acetamidopyridin-3-yl)methyl)-N-(4-((4-methylpiperazin-1-yl)methyl)-3-(trifluoromethyl)phe nyl)indoline-6-carboxamide |
| 14 | 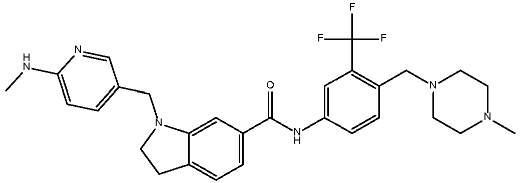 | 1-((6-(methylamino)pyridi n-3-yl)methyl)-N-(4-((4-methylpiperazin-1-yl)methyl)-3-(trifluoromethyl)phe nyl)indoline-6-carboxamide |
| 15 | 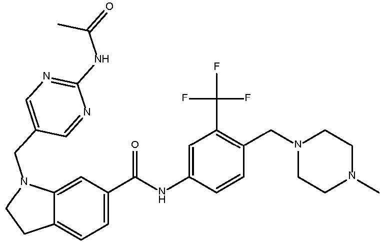 | 1-((2-acetamidopyrimidin-5-yl)methyl)-N-(4-((4-methylpiperazin-1-yl)methyl)-3-(trifluoromethyl)phe nyl)indoline-6-carboxamide |
| 17 | 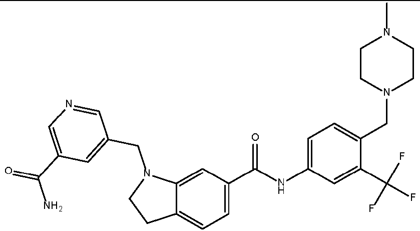 | 1-((5-carbamoylpyridin-3-yl)methyl)-N-(4-((4-methylpiperazin-1-yl)methyl)-3-(trifluoromethyl)phe nyl)indoline-6-carboxamide |

In another particularly preferred embodiment the present invention refers to a compound of formula (I) wherein L₁ is -NHC(O)-, **L** is -CH₂-, represented by formula (Ib) 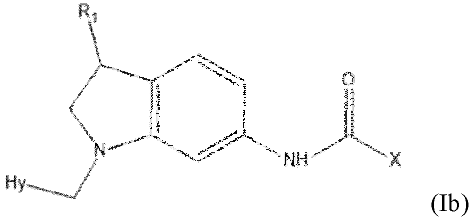 wherein **Hy, R₁** and **X** are as defined above.

In an even more particularly preferred embodiment the present invention refers to a compound of formula (Ib), wherein **X** is 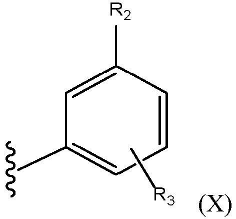 represented by the formula (Ib1) 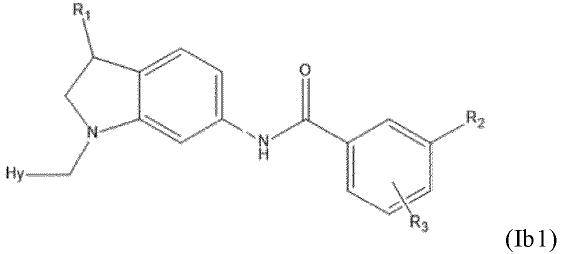 wherein **R₁, Hy, R₂** and **R₃** are as defined above

In a even more preferred embodiment the present invention refers to a compound of formula (I), wherein **L₁ is** -NHC(O)-, **L** is -CH₂-, X is X' 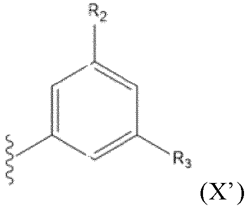 represented by the formula (Ib1') 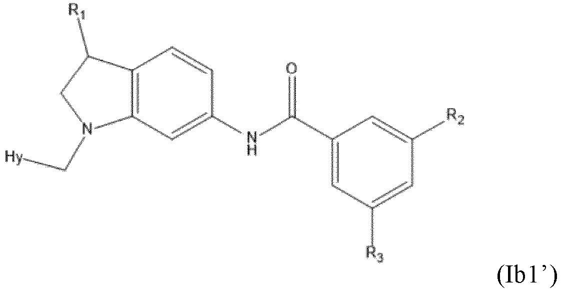 wherein R₁, Hy, R₂ and R₃ are as defined above.

In another even more preferred embodiment the present invention refers to a compound of formula (Ib 1'), wherein R₂ is -CF₃, R₃ is (C₄-C₇)heterocycloalkyl-(C₁-C₄)alkylene-, wherein said -(C₄-C₇)heterocycloalkyl is optionally substituted by one or more -(C₁-C₄)alkyl.

In a preferred embodiment, the invention refers to a compound of general formula (Ib1') wherein R₃ is selected from the group consisting of 4-methylpiperazin-1-yl)methyl and -(4-methyl-1H-imidazol-1-yl).

In a further preferred embodiment, the invention refers to a compound of general formula (Ib1') wherein **Hy** is selected from the group consisting of 3-aminopyrazin-2-yl, 2-((1-methyl-1H-pyrazol-4-yl)amino)pyrimidin-5-yl, 2-aminopyrimidin-5-yl and -N-methyl-4-picolinamide.

According to a preferred embodiment, the invention refers to at least one of the compounds of formula (Ib 1') listed in the Table 3 below and pharmaceutically acceptable salts thereof. These compounds are active on receptors DDR1 and DDR2, as shown in Table 5.

**Table 3: List of compounds of formula (Ib1')**

| **Ex. No.** | **Structure** | **Chemical Name** |
|---|---|---|
| 4 | 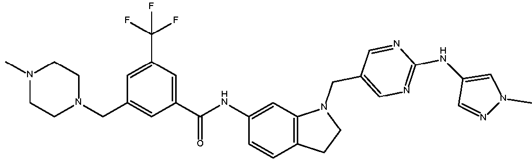 | N-(1-((2-((1-methyl-1H-pyrazol-4-yl)amino)pyrimidin-5-yl)methyl)indolin-6-yl)-3-((4-methylpiperazin-1-yl)methyl)-5-(trifluoromethyl)ben zamide |
| 5 | 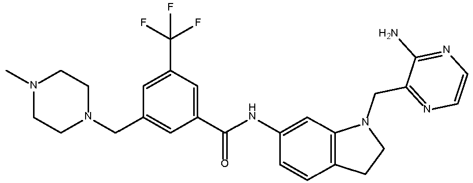 | N-(1-((3-aminopyrazin-2-yl)methyl)indolin-6-yl)-3-((4-methylpiperazin-1-yl)methyl)-5-(trifluoromethyl)ben zamide |
| 6 | 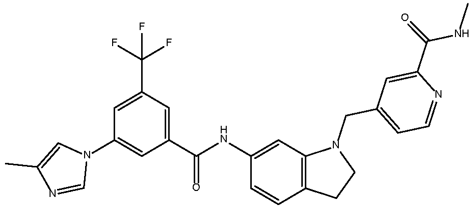 | N-methyl-4-((6-(3-(4-methyl-1H-imidazol-1-yl)-5-(trifluoromethyl)benz amido)indolin-1-yl)methyl)picolinami de |
| 21 | 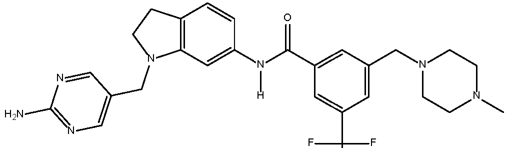 | N-(1-((2-aminopyrimidin-5-yl)methyl)indolin-6-yl)-3-((4-methylpiperazin-1-yl)methyl)-5-(trifluoromethyl)benz amide |

The compounds of the invention, including all the compounds here above listed, can be prepared from readily available starting materials using the following general methods and procedures or by using slightly modified processes readily available to those of ordinary skill in the art. Although a particular embodiment of the present invention may be shown or described herein, those skilled in the art will recognize that all embodiments or aspects of the present invention can be obtained using the methods described herein or by using other known methods, reagents and starting materials. When typical or preferred process conditions (i.e. reaction temperatures, times, mole ratios of reactants, solvents, pressures, etc.) are given, other process conditions can also be used unless otherwise stated. While the optimum reaction conditions may vary depending on the particular reactants or solvent used, such conditions can be readily determined by those skilled in the art by routine optimization procedures.

In some cases, generally known protective groups (PG) could be employed when needed to mask or protect sensitive or reactive moieties, in accordance to general principles of chemistry (Protective group in organic syntheses, 3rd ed. T. W. Greene, P. G. M. Wuts).

The compounds of formula (I) of the present invention have surprisingly been found to effectively inhibit both DDR1 and DDR2. Advantageously, the inhibition of receptors DDR1 and DDR2 may result in efficacious treatment of the diseases or condition wherein the DDR receptors are involved.

In this respect, it has now been found that the compounds of formula (I) of the present invention have an inhibitory potency expressed as Ki constant on DDR1 and DDR2 lower than 80 nM, as shown in the present experimental part. Preferably, the compounds of the present invention have a Ki on DDR1 and DDR2 lower than 50 nM. Even more preferably, the compounds of the present invention have a Ki on DDR1 and DDR2 lower than 25 nM.

In addition, it has been found that the compounds of formula (I) of the present invention have both the affinity for either DDR1 and DD2 receptors and the inhibitory potency against either DDR1 and DDR2 receptors below about 80 nM respectively in the binding (expressed as Ki) and the cell based assays (expressed as IC50), as shown in the present experimental part. Preferably, the compounds of the present invention have a Ki and/or an IC50 on DDR1 and DDR2 receptors lower than 50 nM. Even more preferably, the compounds of the present invention have a Ki and/or an IC50 on DDR1 and DDR2 receptors lower than 25 nM.

In one aspect, the present invention refers to a compound of formula (I) according to any of the embodiments disclosed above for use as a medicament.

In a preferred embodiment, the invention refers to a compound of formula (I) and pharmaceutically acceptable salts thereof, for use in treating diseases, disorders, or conditions associated with dysregulation of DDR.

In another aspect, the invention refers to the use of a compound of formula (I) as above described in the preparation of a medicament for the treatment of disorders associated with dysregulation of DDR.

In a preferred embodiment, the invention refers to a compound of formula (I) or a pharmaceutically acceptable salt thereof, for use in the prevention and/or treatment of a disease, disorder or condition associated with DDR receptor mechanism. In one embodiment, the present invention refers to a compound of formula (I) useful for the prevention and/or treatment of fibrosis and/or diseases, disorders, or conditions that involve fibrosis.

The terms "fibrosis" or "fibrosing disorder," as used herein, refers to conditions that are associated with the abnormal accumulation of cells and/or fibronectin and/or collagen and/or increased fibroblast recruitment and include but are not limited to fibrosis of individual organs or tissues such as the heart, kidney, liver, joints, lung, pleural tissue, peritoneal tissue, skin, cornea, retina, musculoskeletal and digestive tract.

Preferably, the compounds of formula (I) as above described are useful for the treatment and/or prevention of fibrosis such as pulmonary fibrosis, idiopathic pulmonary fibrosis (IPF), hepatic fibrosis, renal fibrosis, ocular fibrosis, cardiac fibrosis, arterial fibrosis and systemic sclerosis.

More preferably, the compounds of formula (I) as above described are for the treatment of idiopathic pulmonary fibrosis (IPF).

In one aspect, the invention also refers to a compound of formula (I) for use in a method for the prevention and/or treatment of disorders associated with DDR receptors mechanisms, said method comprises administering to a patient in need of such treatment a therapeutically effective amount of a compound of formula (I) as above described.

In a further aspect, the invention refers to a compound of formula (I) as above described for use in the treatment of disorders associated with DDR receptors mechanism.

In another aspect, the invention refers to the use of a compound of formula (I) as above described in the preparation of a medicament for the treatment of disorders associated with DDR receptors mechanism.

In a further aspect, the invention refers to a compound of formula (I) for use in a method for the prevention and/or treatment of disorder or condition associated with dysregulation of DDR receptors 1 and 2, said method comprising administering a patient in need of such treatment a therapeutically effective amount of a compound of formula (I) as above described.

In a further aspect, the present invention refers to the compound of formula (I) as above described for use in the treatment of a disease, disorder or condition associated with dysregulation of DDR receptors 1 and 2.

As used herein, "safe and effective amount" in reference to a compound of formula (I) or a pharmaceutically acceptable salt thereof or other pharmaceutically-active agent means an amount of the compound sufficient to treat the patient's condition but low enough to avoid serious side effects and it can nevertheless be routinely determined by the skilled artisan.

The compounds of formula (I) may be administered once or according to a dosing regimen wherein a number of doses are administered at varying intervals of time for a given period of time. Typical daily dosages may vary depending upon the route of administration chosen.

The present invention also refers to a pharmaceutical composition comprising a compound of formula (I) according to any of its embodiment in admixture with at least one or more pharmaceutically acceptable carrier or excipient.

In one embodiment, the invention refers to a pharmaceutical composition of compounds of formula (I) in admixture with one or more pharmaceutically acceptable carrier or excipient, for example those described in Remington's Pharmaceutical Sciences Handbook, XVII Ed., Mack Pub., N.Y., U.S.A.

Administration of the compounds of the invention and their pharmaceutical compositions may be accomplished according to patient needs, for example, orally, nasally, parenterally (subcutaneously, intravenously, intramuscularly, intrasternally and by infusion) and by inhalation.

Preferably, the compounds of the present invention are administered orally or by inhalation.

In one preferred embodiment, the pharmaceutical composition comprising the compound of formula (I) is a solid oral dosage form such as tablets, gelcaps, capsules, caplets, granules, lozenges and bulk powders.

In one embodiment, the pharmaceutical composition comprising the compound of formula (I) is a tablet.

The compounds of the invention can be administered alone or combined with various pharmaceutically acceptable carriers, diluents (such as sucrose, mannitol, lactose, starches) and known excipients, including suspending agents, solubilizers, buffering agents, binders, disintegrants, preservatives, colorants, flavorants, lubricants and the like.

In a further embodiment, the pharmaceutical composition comprising a compound of formula (I) is a liquid oral dosage forms such as aqueous and non-aqueous solutions, emulsions, suspensions, syrups, and elixirs. Such liquid dosage forms can also contain suitable known inert diluents such as water and suitable known excipients such as preservatives, wetting agents, sweeteners, flavorants, as well as agents for emulsifying and/or suspending the compounds of the invention.

In a further embodiment, the pharmaceutical composition comprising the compound of formula (I) is an inhalable preparation such as inhalable powders, propellant-containing metering aerosols or propellant-free inhalable formulations.

For administration as a dry powder, single- or multi-dose inhalers known from the prior art may be utilized. In that case the powder may be filled in gelatine, plastic or other capsules, cartridges or blister packs or in a reservoir.

A diluent or carrier chemically inert to the compounds of the invention, e.g. lactose or any other additive suitable for improving the respirable fraction may be added to the powdered compounds of the invention.

Inhalation aerosols containing propellant gas such as hydrofluoroalkanes may contain the compounds of the invention either in solution or in dispersed form. The propellant-driven formulations may also contain other ingredients such as co-solvents, stabilizers and optionally other excipients.

The propellant-free inhalable formulations comprising the compounds of the invention may be in form of solutions or suspensions in an aqueous, alcoholic or hydroalcoholic medium and they may be delivered by jet or ultrasonic nebulizers known from the prior art or by soft-mist nebulizers.

The compounds of the invention can be administered as the sole active agent or in combination with other pharmaceutical active ingredients.

The dosages of the compounds of the invention depend upon a variety of factors including among others the particular disease to be treated, the severity of the symptoms, the route of administration and the like.

The invention is also directed to a device comprising a pharmaceutical composition comprising a compound of formula (I) according to the invention, in form of a single- or multi-dose dry powder inhaler or a metered dose inhaler.

All preferred groups or embodiments described above for compounds of formula (I) may be combined among each other and apply as well *mutatis mutandis.*

The compounds of the invention, including all the compounds here above listed, can be prepared from readily available starting materials using the following general methods and procedures or by using slightly modified processes readily available to those of ordinary skill in the art. Although a particular embodiment of the present invention may be shown or described herein, those skilled in the art will recognize that all embodiments or aspects of the present invention can be obtained using the methods described herein or by using other known methods, reagents and starting materials. When typical or preferred process conditions (i.e. reaction temperatures, times, mole ratios of reactants, solvents, pressures, etc.) are given, other process conditions can also be used unless otherwise stated. While the optimum reaction conditions may vary depending on the particular reactants or solvent used, such conditions can be readily determined by those skilled in the art by routine optimization procedures.

Thus, processes described below and reported in the following Schemes should not be viewed as limiting the scope of the synthetic methods available for the preparation of the compounds of the invention.

The compounds of formula (I) including all the compounds or at least one of the here above listed can be generally prepared according to the procedure outlined in detail in the Schemes shown below, using generally known methods.

Compounds of formula (I) wherein R₁, L, L₁, Hy and X are as defined above, may be prepared according to Scheme 1 as described hereinafter providing at least one non-limiting synthetic route for the preparation of all examples.

In a first embodiment of the present invention, compounds of formula (I) can be prepared as described in Scheme 1.

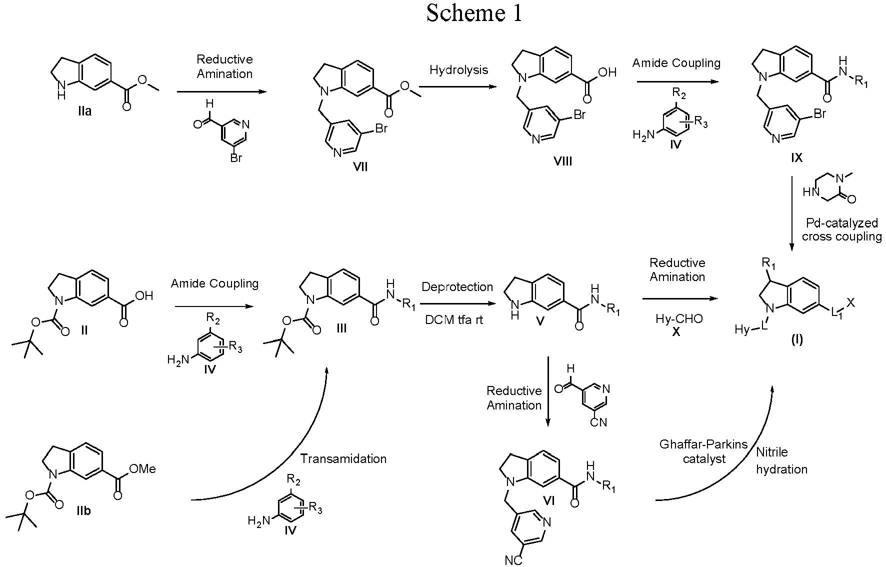

According to Scheme 1, Intermediate III may be prepared following a one-step synthesis starting from intermediate II, under suitable amide coupling reaction conditions. For example, intermediate II and IV may be reacted in the presence of an agent, such as T3P or HATU, that activates the carboxylic acid partner for subsequent reaction with amines; with an organic base, such as DIPEA or TEA, in a suitable organic solvent, such as DCM or DMF, and at a temperature generally around RT for a time ranging from a few hours to overnight. Alternatively, Intermediate III may be prepared via amidation starting from intermediate II in the presence of TCFH and 1-methylimidazole to give the transient activated acylimidazolinium intermediate that reacted with the appropriate amine IV in a solvent such as DMF, at a suitable temperature, for example room temperature.

In a different approach, direct amidation of esters (transamidation) may be carried on between intermediate IIb and Intermediate IV to obtain Intermediate III, using for example butyllithium as a promoter in a suitable organic solvent as THF or Dioxane at - 78°C.

The intermediate III can be converted into intermediate V by deprotection of a BOC-protected amine under acidic conditions such as trifluoroacetic acid in a suitable solvent, such as, but not limited to, DCM at room temperature for few hours. Reductive amination of Intermediate V with the appropriate aldehyde Hy-CHO X, to afford compounds of formula (I), may be carried out with a suitable reductant agent, such as Na(OAc)₃BH or NaCNBH₃, in a suitable solvent, such as DCM or EtOH, in presence of an acid, such as acetic acid, and a coordinating agent, such as titanium tetrahydroisopropoxide, if needed, at room temperature.

Alternatively, a reductive amination may be carried out to convert Intermediate V to Intermediate VI, with the proper aldehyde, such as 5-formylnicotinonitrile, following the conditions described above, followed by nitrile hydration conditions that may be applied to intermediate VI using the appropriate hydrating agent, such as Ghaffar-Parkins catalyst, in a suitable solvent, such as water, at the appropriate temperature, for example 100 °C, to obtain Compounds of formula (I).

Differently, intermediate IIa can be converted into the intermediate VII performing a reductive amination with a proper aldehyde, such as 5-bromonicotinaldehyde, using the conditions described above. Intermediate VII may undergo hydrolysis applying the conditions described above, to give intermediate VIII, that can be converted into intermediate IX performing an amide coupling, applying the conditions described above.

Finally, Compounds of formula (I) can be prepared from intermediate IX carrying out a Pd-catalyzed cross coupling with the proper amine, such as 1-methylpiperazin-2-one, using the appropriate Pd-ligand system, such as RuPhos Pd G3, in a suitable base, such as cesium carbonate or potassium carbonate, in a suitable solvent, such as dioxane or DMF, at the proper temperature, for example 80 °C.

Compounds of formula (I) may be prepared according to Scheme 2 as described hereinafter providing at least one non-limiting synthetic route for the preparation of all examples.

In another embodiment, compounds of formula (I) wherein R₁, L, L₁, Hy and X are as defined above, can be prepared as described in Scheme 2.

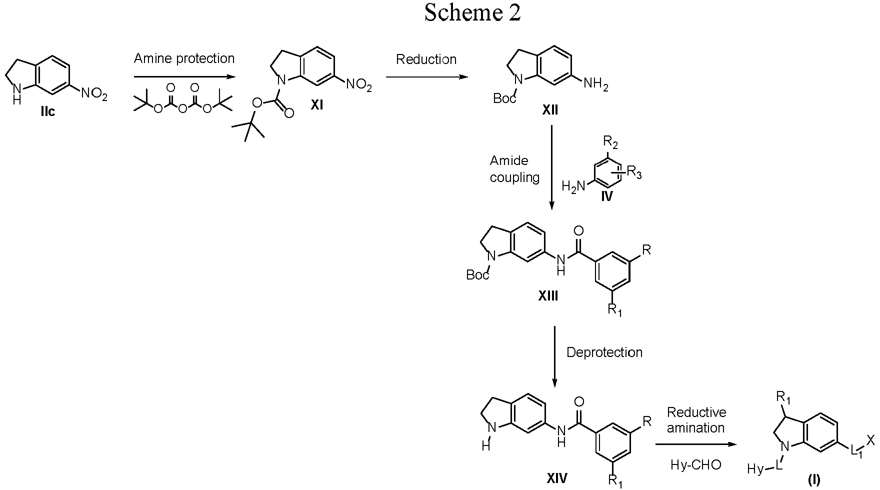

According to Scheme 2, Intermediate XI may be prepared starting from intermediate IIc in the presence of BOC-Anhydride and DMAP with an organic base such as DIPEA or TEA, in a suitable organic solvent, such as DCM, and at a temperature generally around room temperature for a time ranging from a few hours to overnight. The Nitro group of intermediate XI can be reduced to the corresponding amine of intermediate XII by reduction under hydrogen atmosphere in presence of a suitable catalyst such as Pd/C in a suitable solvent such as, but not limited to, ethyl acetate at room temperature for few hours. Intermediate XIII may be prepared under suitable amide coupling reaction conditions from intermediate XII. For example, intermediate XII and IV may be reacted in the presence of an agent, such as HATU or TBTU, that activates the carboxylic acid partner for subsequent reaction with amines, with an organic base, such as DIPEA or TEA, in a suitable organic solvent such as DCM or DMF, and at a temperature generally around room temperature for a time ranging from a few hours to overnight. The intermediate XIII can be converted into intermediate XIV by deprotection of a BOC-protected amine under acidic conditions such as trifluoroacetic acid in a suitable solvent such as, but not limited to, DCM at room temperature for few hours. Reductive amination of Intermediate XIV with the appropriate aldehyde Hy-CHO, to afford compounds of formula (I), may be carried out with a suitable reducing agent, such as Na(OAc)₃BH or NaCNBH₃, in a suitable solvent, such as DCM or EtOH, in presence of an acid, such as acetic acid, if needed, and a coordinating agent, such as titanium tetrahydroisopropoxide, if needed, at a proper temperature, for example room temperature.

The various aspects of the invention described in this application are illustrated by the following examples which are not meant to limit the invention in any way.

### PREPARATIONS OF INTERMEDIATES AND EXAMPLES

Chemical Names of the compounds were generated with Structure To Name Place IUPAC Name by PerkinElmer ChemDraw Professional 19.1.1.21. All reagents, for which the synthesis is not described in the experimental part, are either commercially available, or are known compounds or may be formed from known compounds by known methods by a person skilled in the art.

In the procedures that follow, some of the starting materials are identified through an "Intermediate" or "Example" number with indications on step number. This is provided merely for assistance to the skilled chemist.

A "similar" or "analogous" procedure means that such a procedure may involve minor variations, for example reaction temperature, reagent/solvent amount, reaction time, work-up conditions or chromatographic purification conditions.

### ABBREVIATION - MEANING

t*_{R}* = retention time; (CH₃)₃COK = potassium tert-butylate; Boc = tert-butoxycarbonyl; TEA = triethylamine; DMF = dimethylformamide; EtOAc = Ethyl acetate; RT or rt = room temperature; THF = tetrahydrofuran; DCM = dichloromethane; MeOH = methyl alcohol; LCMS = Liquid Chromatography/Mass Spectrometry; HPLC = high performance liquid chromatography; d-DMSO = deuterated dimethyl sulfoxide. NMR = nuclear magnetic resonance; DIPEA = N,N-Diisopropylethylamine; UPLC = Ultra Performance Liquid Chromatography; tBu XPhos = 2-Di-tert-butylphosphino-2',4',6'-triisopropylbiphenyl; Pd(dba)₂ = Bis(dibenzylideneacetone)palladium(0); PdCl₂(dppf) = [1,1'-Bis(diphenylphosphino)ferrocene]dichloro palladium(II); RuPhos = 2-dicyclohexylphosphino-2',6'-diisopropoxy-1,1'-biphenyl; STAB = sodium triacetoxyborohydride; AcOH = Acetic acid; prepHPLC = preparative high performance liquid chromatography; NaBH₃CN =sodium cyanoborohydride; Na₂SO₄ = sodium sulfate; FCC= flash column chromatography; BuLi= nButyllithium; NaHCO₃ = 15 % or saturated solution (sat. aq. sol.) of sodium bicarbonate; SM= starting material; SCX = strong cation exchange; TFA = trifluoroacetic acid; T3P = Propylphosphonic anhydride; sat.sol. = saturated solution.

### General Experimental details and methods

### NMR characterization:

¹H NMR spectra were recorded on Varian MR-400 spectrometer operating at 400 MHZ (proton frequency), equipped with: a self-shielded Z-gradient coil 5 mm 1H/nX broadband probe head for reverse detection, deuterium digital lock channel unit, quadrature digital detection unit with transmitter offset frequency shift, alternatively ¹H NMR spectra were recorded on Bruker Fourier 300 MHz. Chemical shifts are reported as δ values in ppm relative to tetramethyl silane (TMS) as an internal standard. Coupling constants (J values) are given in hertz (Hz) and multiplicities are reported using the following abbreviation (s= singlet, d=doublet, t=triplet, q=quartet, m=multiplet, br=broad, nd=not determined).

In some cases, signals NH from amide bond or amine bond (Exchangeable protons) are not visible.

In a few cases, signals of CH₂ of indoline ring could be hidden under the signal of water or under the signal of DMSO.

### LC/UV/MS Analytical Methods

LC/MS retention times are estimated to be affected by an experimental error of ± 0.5 min.

Method 1: Kinetex^{®} XB-C18 column, 4.6x50 mm, 2.6 µm maintained at 25 °C. Mobile phase: water (0.1% formic acid) in MeCN (0.1% formic acid), from 80% to 5% within 3.90 min; Flow rate: 1.0 ml/min; wavelength: 190-340 nm DAD. Dionex UHPLC Ultimate 3000 with DAD detector/Thermo Scientific MSQ Plus.

Method 2: Acquity CSH C18 column 50mm x 2.1mm 1.7µm, maintained at 40°C; Mobile Phase: Eluent B (MeCN/water 95:5 +0.05% HCOOH) in Eluent A (water/MeCN 95:5 +0.05% HCOOH) from 1% to 99.9% within 1.5 min. Flow rate: 1 mL/min. Wavelength: 210-400 nm DAD. UPLC ± Waters PDA ± Waters QDA.

Method 3: Acquity UPLC HSS C18 column, 100 x 2.1mm, 1.8 µm (Plus guard cartridge), maintained at 40°C. Mobile phase: MeCN (0.1% formic acid) in water (0.1% formic acid) from 5% to 95% within 5.6 min. Flow rate: 0.4 ml/min. Wavelength: 210-400 nm DAD. UPLC + Waters DAD + Waters SQD2, single quadrapole UPLC-MS

Method 4: Kinetex^{®} XB-C18 column, 4.6x50 mm, 2.6 µm maintained at 25 °C. Mobile phase: water (0.1% formic acid) in ACN (0.1% formic acid), from 80% to 5% within 3.90 min; Flow rate: 1.0 ml/min; wavelength: 190-340 nm DAD. Dionex UHPLC Ultimate 3000 with DAD detector/Thermo Scientific MSQ Plus.

Method 5: Acquity BEH UPLC column, 2.1x50mm, 1.7µm, maintained at 40°C. Mobile phase: MeCN (0.03% ammonia) in water (0.03% ammonia), from 8% to 97% within 1.5 min; Flow rate: 0.8 ml/min; Wavelength: 210-400 nm DAD. Acquity H-Class UPLC with PDA detector and QDa.

Where the preparation of starting materials is not described, these are commercially available, known in the literature, or readily obtainable by those skilled in the art using standard procedures. All solvents were purchased from commercial sources and were used without additional purification.

Preparative HPLC was performed using reversed phase (C18) preparative HPLC either in basic conditions (ACN+0.1%NH₃, H₂O+0.1%NH₃ or ACN, H₂O ± 0.05% NH₃ or in acidic conditions (ACN+0.1%FA, H₂O+0.1%FA); in the latter case, SCX (NH) was utilized to obtained free base of the product, unless differently stated.

Flash chromatography (FCC) was performed on Interchim PuriFlash 450 and 520Plus systems using pre-packed silica gel cartridges or Isolera^{™} flash purification system.

Thin layer chromatography was performed on Merck silica gel 60 F254 TLC plates. Preparative thin-layer chromatography (pTLC) was performed with Uniplate 1000 micron or 500 micron silica gel plates.

The starting material may not necessarily have been prepared from the batch referred to.

When reference is made to the use of a "similar" or "analogous" procedure, as will be appreciated by those skilled in the art, such a procedure may involve minor variations, for example reaction temperature, reagent/solvent amount, reaction time, work-up conditions or chromatographic purification conditions. All final compounds were obtained as a free base, unless stated otherwise.

### PREPARATION OF INTERMEDIATES

### Preparation of 3-((4-methylpiperazin-1-yl)methyl)-5-(trifluoromethyl)benzoic acid - Intermediate 1

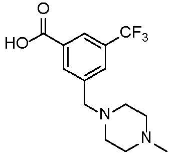

### Step 1; methyl 3-bromo-5-(trifluoromethyl)benzoate - Intermediate 2

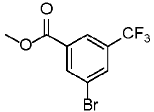

To the solution of 3-bromo-5-(trifluoromethyl)benzoic acid (75.0 g, 279 mmol) in MeOH (282 mL), SOCl₂ (81.0 mL, 1115 mmol) was added dropwise at 0°C. Next, the reaction mixture was stirred under reflux overnight, whereupon volatiles were removed in vacuum. To the residue water (200 mL) was added and aqueous layer was extracted with EtOAc (2x250 mL). Combined organic layers were washed with saturated solution of NaHCO₃, dried over Na₂SO₄ concentrated under vacuum to afford the title product (74.5 g, 94%).

¹H NMR (300 MHz, DMSO-d6) δ 8.30 (dt, J = 1.8, 0.8 Hz, 2H), 8.13 (td, J = 1.6, 0.8 Hz, 1H), 3.90 (s, 3H).

### Step 2; methyl 3-[(4-methylpiperazin-1-yl)methyl]-5-(trifluoromethyl) benzoate - Intermediate 3

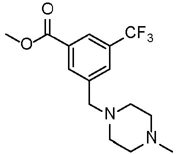

Intermediate 2 (47.5 g, 168 mmol), Cs₂CO₃ (164 g, 503 mmol), potassium 1-methyl-4-trifluoroboratomethylpiperazine (40.6 g, 184.6 mmol) were suspended in a mixture of THF (100 mL) and water (11 mL). The suspension was degassed, then Pd(OAc)₂ (3.76 g, 16.8 mmol) and XPhos (16.0 g, 33.5 mmol) were added and the reaction was carried out at 80 °C for 24 h. The reaction mixture was diluted with water (100 mL) and extracted with EtOAc (2x150 mL). The combined organic phases were concentrated and dried under vacuum to afford the crude, which was purified by column chromatography (DCM:MeOH, 9:1) to give the title compound (25.3 g, 48%).

¹H NMR (300 MHz, DMSO-*d*₆) δ 8.16 (d, *J* = 1.7 Hz, 1H), 8.07 (t, *J* = 1.8 Hz, 1H), 7.97 - 7.86 (m, 1H), 3.90 (s, 3H), 3.62 (s, 2H), 2.38 (s, 8H), 2.15 (s, 3H).

### Step 3; 3-((4-methylpiperazin-1-yl)methyl)-5-(trifluoromethyl)benzoic acid - Intermediate 1

Intermediate 3 (25.3 g, 80.0 mmol) was dissolved in MeOH (700 mL). 1M LiOH solution was added (3.8 g, 160 mL) to the reaction mixture and stirred at RT overnight. The solvent was removed under vacuum and the crude material triturated with diethyl ether (2x) and filtered. The solid was collected to give the title compound (26.0 g, 100%).

¹H NMR (300 MHz, DMSO-d6) δ 8.02 (s, 2H), 7.49 (s, 1H), 3.51 (s, 2H), 2.32 (s, 8H), 2.14 (s, 3H).

The salt was dissolved in 2M aqueous HCl till pH 4 then the solution was concentrated under reduced pressure. Residue was washed with water, filtered and dried under vacuum to yield the title compound as corresponding carboxylic acid in quantitative yield.

### Preparation of 2-((1-methyl-1H-pyrazol-4-yl)amino) pyrimidine-5-carbaldehyde - Intermediate 4

### Step 1; 2-((1-methyl-1H-pyrazol-4-yl)amino) pyrimidine-5-carbaldehyde - Intermediate 4

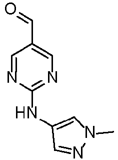

2-chloropyrimidine-5-carbaldehyde (50 mg, 0.351 mmol) was dissolved in THF (3.5 ml) then 1-methyl-1H-pyrazol-4-amine (41 mg, 0.421 mmol), TEA (0.1 ml, 0.719 mmol) and THF (3.5 ml) were added. Reaction mixture was stirred overnight at room temperature. The mixture was quenched with DCM and brine. The phases were separated and the water layer was washed with DCM (x2). The combined organic phases were dried over Na₂SO₄, filtered and concentrated under vacuum. Crude material was purified by FCC eluting with hexane:ethyl acetate 1:1 to give the title product (35 mg, 39%).

¹H NMR (300 MHz, DMSO-d6) δ 10.45 (s, 1H), 9.80 (s, 1H), 8.92 - 8.79 (m, 2H), 7.99 (d, J = 0.8 Hz, 1H), 7.56 (d, J = 0.8 Hz, 1H), 3.83 (s, 3H).

### Preparation of 2-((1-(2-methoxyethyl)-1H-pyrazol-4-yl)amino)pyrimidine-5-carbaldehyde- (Intermediate 17)

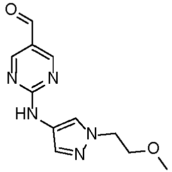

### Step 1; 2-chloro-5-(diethoxymethyl)pyrimidine (Intermediate 18)

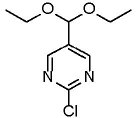

2-chloropyrimidine-5-carbaldehyde (2.07 g, 14.52 mmol), triethyl orthoformate (7.24 ml, 43.5 mmol) and p-toluenesulfonic acid monohydrate (276 mg, 1.451 mmol) were dissolved in EtOH absolute (41 ml) in a dried round bottom flask under Ar. The mixture was stirred at reflux for 1 hr. The solution was cooled with an ice bath. 40 mL of saturated NaHCO₃ were added. The mixture was allowed to warm up to RT. EtOH was evaporated and the resulting suspension was extracted with AcOEt. Organic phases were combined, dried over Na₂SO₄, filtered and evaporated to dryness. Crude material was purified on FCC (100/0 → 80/20 Hexane/AcOEt) to give title compound (2.68 g, 12.37, 85 %)

NMR: ¹H NMR (300 MHz, DMSO-d6) δ 8.75 (s, 2H), 5.68 (s, 1H), 3.67 - 3.50 (m, 4H), 1.17 (t, J = 7.0 Hz, 6H).

### Step 2; 5-(diethoxymethyl)-N-(1-(2-methoxyethyl)-1H-pyrazol-4-yl)pyrimidin-2-amine (Intermediate 19)

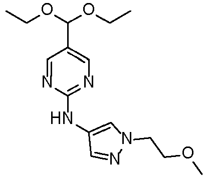

Previous Intermediate 18 (435 mg, 2.008 mmol) was dissolved in toluene (7 ml) and dioxane (7.0 ml) in reaction tube under Ar.1-(2-methoxyethyl)-1H-pyrazol-4-amine (0.3 ml, 2.53 mmol) and potassium phosphate tribasic (1.32 g, 6.22 mmol) were added to the solution. The solution was purged with Ar for 15 min. 2-Dicyclohexylphosphino-2',4',6'-triisopropylbiphenyl (98 mg, 0.206 mmol) and Pd₂(dba)₃ (94 mg, 0.103 mmol) were added to the solution. The tube was sealed and the mixture was heated at 100 °C for 48 hr. The crude mixture was filtered through celite, washed with MeOH and evaporated. After purification by FCC (DCM/MeOH/NH3 (from 100/0 to 90/10), the title compound was obtained (458 mg, 1.42 mmol, 71 %).

The compound reported in the following table was prepared as described for Intermediate 19, applying the corresponding, commercially available amine.

| **Intermediate No** | **Structure** | **Reagents Amount** | **Amount final product (yield)** |
|---|---|---|---|
| 20 | 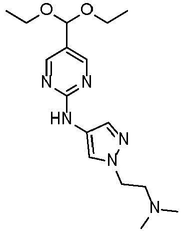 | Amine 1.21 g, 7.92 mmol | 510 mg (19%) |
| | | Intermediate 18: 1.68 g | |

### Step 3; 2-((1-(2-methoxyethyl)-1H-pyrazol-4-yl)amino)pyrimidine-5-carbaldehyde (Intermediate 17)

Intermediate 19 (458 mg, 1.425 mmol) was placed in a round-bottom flask. Hydrochloric acid, 1N in water (45 ml, 45.0 mmol) was added and the mixture was stirred overnight at RT. The mixture was cooled with ice bath and saturated NaOH solution was slowly added until pH=13. The aqueous phase was extracted with AcOEt. Organic layer was dried over Na₂SO₄ and evaporated to give title compound (312 mg, 1.62 mmol, 89 %).

¹H NMR (300 MHz, DMSO-d6) δ 10.44 (s, 1H), 9.80 (s, 1H), 8.92 - 8.80 (m, 2H), 8.01 (s, 1H), 7.60 (s, 1H), 4.24 (t, J = 5.3 Hz, 2H), 3.66 (t, J = 5.3 Hz, 2H), 3.23 (s, 3H).

The compound reported in the following table was prepared as described for Intermediate 17, applying the corresponding, commercially available amine.

| **Intermediate No** | **Structure** | **Reagents Amount** | **Amount final product (yield)** | **Data** |
|---|---|---|---|---|
| 21 | 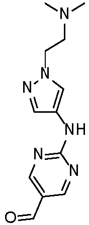 | Intermediate 20 510 mg | 349 mg (89%) | ¹H NMR (300 MHz, DMSO-d6) δ : 10.43 (s, 1H), 9.80 (s, 1H), 8.85 (dd, J = 20.2, 2.2 Hz, 2H), 8.02 (s, 1H), 7.58 (s, 1H), 4.17 (t, J = 6.5 Hz, 2H), 2.62 (t, J = 6.5 Hz, 2H), 2.16 (s, 6H) |

### Preparation of N-(5-formylpyrimidin-2-yl)acetamide - (Intermediate 22)

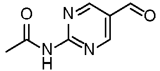

### Step 1; N-(5-formylpyrimidin-2-yl)acetamide (Intermediate 22)

2-Aminopyrimidine-5-carboxaldehyde (100 mg, 0.812 mmol) was dissolved in acetic anhydride (2.0 mL, 22.8 mmol) and the reaction mixture was heated at 140°C for 90 min. The reaction mixture was allowed to cool to RT and the solid, precipitated adding 2:1 cyclohexane/diethyl ether, was filtered, washed with cyclohexane and dried in vacuo. To afford title compound (84 mg, 0.5086 mmol, 62.6% yield).
¹H NMR (400 MHz, CDCl₃) d 10.03 (s, 1H), 9.04 (s, 2H), 8.76 (s, 1H), 2.58 (s, 3H).

### PREPARATIONS OF EXAMPLES

### Example 1: Preparation of N-(3-(4-methyl-1H-imidazol-1-yl)-5-(trifluoromethyl)phenyl)-1-(pyrimidin-5-ylmethyl)indoline-6-carboxamide)

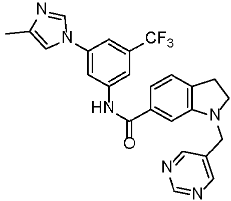

### Example 1

### Step 1; tert-butyl 6-((3-(4-methyl-1H-imidazol-1-yl)-5-(trifluoromethyl) phenyl)carbamoyl)indoline-1-carboxylate - Intermediate 5

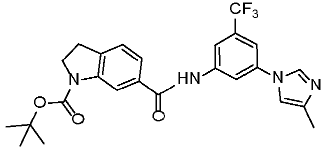

1-(tert-butoxycarbonyl)indoline-6-carboxylic acid (1 g, 3.80 mmol) and 3-(4-methyl-1H-imidazol-1-yl)-5-(trifluoromethyl)aniline (0.916 g, 3.80 mmol) were dissolved in DCM (2 ml). Next, DIPEA (1.990 ml, 11.39 mmol) and 50% T3P in ethyl acetate (4.48 ml, 7.60 mmol) were added. Reaction mixture was stirred at 20 °C over 20 hr. Reaction mixture was diluted with DCM (10 ml), next water (5ml) was added. Phases were separated, organic layer was washed with citric acid (5% aq.sol, 2x10 ml), dried over Na₂SO₄, filtered and concentrated to dryness. Crude material was purified via column chromatography eluting with DCM/MeOH, starting from 100% of DCM to 5% of MeOH in DCM. Only 200 mg of pure desired product was obtained and 1.4 g of mix of desired product and starting material (acid) was obtained. This material was dissolved in DCM (100 ml) and washed Na₂CO₃ (3x50 ml) and brine (100 ml) to remove residual of acid. Organic layer was dried over Na₂SO₄, filtered, evaporated to dryness on rotatory evaporator giving the title compound (1.01 g, 55 %).

¹H NMR (300 MHz, DMSO-d6) δ 10.66 (s, 1H), 8.28 (d, J = 2.4 Hz, 1H), 8.20 (d, J = 1.4 Hz, 1H), 8.14 (s, 1H), 7.71 (d, J = 2.1 Hz, 1H), 7.58 (dd, J = 7.7, 1.7 Hz, 1H), 7.49 (t, J = 1.3 Hz, 1H), 7.37 (d, J = 7.8 Hz, 1H), 3.98 (t, J = 8.7 Hz, 2H), 3.15 (t, J = 8.7 Hz, 2H), 2.18 (d, J = 1.0 Hz, 3H), 1.53 (s, 8H).

### Step 2; N-(3-(4-methyl-1H-imidazol-1-yl)-5-(trifluoromethyl)phenyl) indoline-6-carboxamide - Intermediate 6

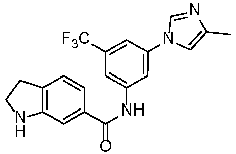

Intermediate 5 (1.01 g, 2.076 mmol) was dissolved in DCM (5 ml) then trifluoroacetic acid (1.440 ml, 18.68 mmol) was added. Reaction mixture was stirred at RT overnight. Reaction mixture was diluted with DCM (10 ml), NaHCO₃ (sat. aq. sol.) was added. Mixture was left for stirring for 30 minutes at room temperature (bubbling was observed). Next layers were partitioned. Organic layer was washed with NaHCO₃, dried over Na₂SO₄ and evaporated under vacuum. Material was used further without additional purification. The title compound, as a free base, was obtained (900 mg, 100 %).

¹H NMR (300 MHz, DMSO-d6) δ 10.49 (s, 1H), 8.31 (dd, J = 4.2, 2.1 Hz, 2H), 8.17 (d, J = 1.7 Hz, 1H), 7.71 (d, J = 1.8 Hz, 1H), 7.53 (d, J = 1.4 Hz, 1H), 7.25 - 7.13 (m, 2H), 7.06 (d, J = 1.3 Hz, 1H), 3.47 (d, J = 8.5 Hz, 3H), 2.98 (t, J = 8.5 Hz, 2H), 2.20 (d, J = 1.0 Hz, 3H).

### Step 3; N-(3-(4-methyl-1H-imidazol-1-yl)-5-(trifluoromethyl)phenyl)-1-(pyrimidin-5-ylmethyl)indoline-6-carboxamide (Example 1)

Intermediate 6 (0.1 g, 0.259 mmol) was dissolved in DCM (2 ml) then pyrimidine-5-carbaldehyde (0.028 g, 0.259 mmol), NaCNBH₃ (0.024 g, 0.388 mmol) and AcOH (0.030 ml, 0.518 mmol) were added. Reaction mixture was stirred 18 hr at 20 °C. Reaction mixture was evaporated, then diluted with DCM (5 ml), next water (5 ml) was added. Layers were partitioned, organic layer was washed with K₂CO₃ (aq.sat.sol.) 2x10 ml, dried over Na₂SO₄, filtered and concentrated under reduced pressure. Crude material was purified via preparative HPLC eluting with ACN+0.1%NH₃, H₂O+0.1%NH₃, then the residue was repurified via preparative TLC eluting with DCM/MeOH, 9:1 to provide the title compound (14 mg, 11 %).

| **Example No.** | **Analytical data** |
|---|---|
| 1 | ¹H NMR (300 MHz, DMSO-d6) δ 10.51 (s, 1H), 9.14 (s, 1H), 8.84 (s, 2H), 8.28 (t, J = 2.0 Hz, 1H), 8.20 (d, J = 1.4 Hz, 1H), 8.13 (s, 1H), 7.70 (s, 1H), 7.48 (t, J = 1.4 Hz, 1H), 7.32 (dd, J = 7.6, 1.4 Hz, 1H), 7.23 (dd, J = 4.7, 3.1 Hz, 2H), 4.45 (s, 2H), 3.38 (d, J = 3.5 Hz, 2H), 3.01 (t, J = 8.3 Hz, 2H), 2.18 (d, J = 1.0 Hz, 3H). LC-MS (ESI): (Method 1) t*_{R} =* 1.85 min; m/z (M+1)=478.97 |

**Example 2** was prepared via reductive amination as described for **Example 1,** step 1-3, applying the corresponding, commercially available carbaldehyde in step 3:

| **Ex. No.** | **Structure** | **Amount SM** | **Product Amount (Yield)** | **Purification method** | **Analytical data** |
|---|---|---|---|---|---|
| 2 | 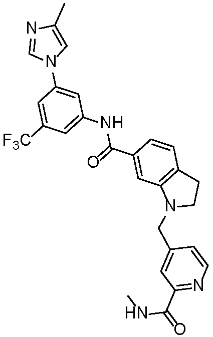 | 0.10 | 0.010 g (Y=8%) | pTLC | ¹H NMR (300 MHz, DMSO-d6) δ 10.45 (s, 1H), 8.79 (d, J = 5.1 Hz, 1H), 8.60 (d, J = 4.9 Hz, 1H), 8.26 (d, J = 2.1 Hz, 1H), 8.19 (d, J = 1.5 Hz, 1H), 8.10 (s, 1H), 8.03 (s, 1H), 7.70 (s, 1H), 7.57 (dd, J = 5.0, 1.7 Hz, 1H), 7.47 (s, 1H), 7.32 (dd, J = 7.6, 1.4 Hz, 1H), 7.25 (d, J = 7.6 Hz, 1H), 7.07 (d, J = 1.5 Hz, 1H), 4.51 (s, 2H), 3.44 (t, J = 8.4 Hz, 2H), 3.06 (t, J = 8.4 Hz, 2H), 2.81 (d, J = 4.8 Hz, 3H), 2.17 (d, J = 1.0 Hz, 3H). |
| | | | | | LC-MS (ESI): (Method 1) |
| | | | | | t*_{R}* =2.08 min; m/z (M+1)=534.98 |

### Example 3: Preparation of N-(3-((4-methylpiperazin-1-yl)methyl)-5-(trifluoromethyl)phenyl)-1-(pyrimidin-5-ylmethyl)indoline-6-carboxamide

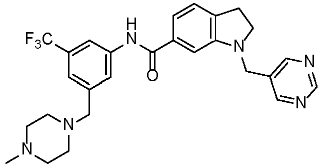

### Example 3

### Step 1; tert-butyl 6-((3-((4-methylpiperazin-1-yl)methyl)-5-(trifluoromethyl) phenyl)carbamoyl)indoline-1-carboxylate - Intermediate 7

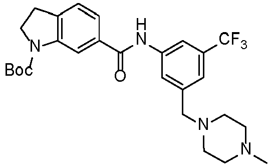

In a dried (heat gun/vacuum/N₂ cycles), double-necked 25mL flask, 3-((4-methylpiperazin-1-yl)methyl)-5-(trifluoromethyl)aniline (0.542 g, 1.983 mmol) was dissolved in anhydrous THF (15 ml) and cooled down to -78 °C, then butyllithium (0.793 ml, 1.983 mmol) was added dropwise. The solution was stirred for 15 minutes, then a solution of 1-(tert-butyl) 6-methyl indoline-1,6-dicarboxylate (0.275 g, 0.992 mmol) in anhydrous THF (1 mL) was added dropwise. The mixture was stirred for 16h while temperature raised from -78 °C up to room temperature. Water (50 mL) was added, then extraction with EtOAc (3x10 mL) was done. The organic phase was washed with brine (30mL), dried over Na₂SO₄, filtered and concentrated to dryness. The crude was purified on FCC (NH) eluting system DCM/MeOH, from 100:0 to 90:10 to afford the title compound (518mg, 100%).

¹H NMR (DMSO-d6, 400MHz): δ = 10.48 (s, 1H), 8.15 (s, 1H), 7.99 (s, 1H), 7.57 (d, 1H, J = 7.7 Hz), 7.36-7.32 (m, 2H), 6.76 (s, 1H), 5.51 (s, 2H), 3.97 (t, 2H, J = 8.7 Hz), 3.14 (t, 2H, J = 8.7 Hz), 2.33 (bs, 8H), 2.15 (s, 3H), 1.53 (s, 9H) ppm

### Step 2; N-(3-((4-methylpiperazin-1-yl)methyl)-5-(trifluoromethyl) phenyl) indoline-6-carboxamide - Intermediate 8

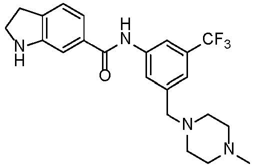

**Intermediate 7** (0.514 g, 0.991 mmol) was dissolved in a 3:1 mixture of DCM (12 ml) and 2,2,2-trifluoroacetic acid (3.04 ml, 39.6 mmol). The solution was stirred at room temperature for 1 h. After evaporation of solvents, crude was diluted with water (20mL) then pH was adjusted to ca 7-8. Product was extracted with DCM (2x20mL) via phase separator. The organic phase was then concentrated to dryness to give the title compound (415mg, 100%).

¹H NMR (DMSO-d₆, 400MHz): δ = 10.25 (s, 1H), 8.13 (s, 1H), 7.96 (s, 1H), 7.28 (s, 1H), 7.15 (d, 1H, J = 7.5 Hz), 7.11 (d, 1H, J = 7.5 Hz), 7.01 (s, 1H), 4.04 (q, 1H, J = 5.1 Hz), 3.49 (s, 2H), 3.44 (t, 2H, J = 8.7 Hz), 2.94 (t, 2H, J = 8.7 Hz), 2.34-2.27 (m, 8H), 2.13 (s, 3H) ppm

### Step 3; N-(3-((4-methylpiperazin-1-yl)methyl)-5-(trifluoromethyl)phenyl)-1-(pyrimidin-5-ylmethyl)inoline-6-carboxamide (Example 3)

In a 40mL vial, equipped with a magnetic stir bar, a solution of **Intermediate 8** (0.05 g, 0.119 mmol) and pyrimidine-5-carbaldehyde (0.013 g, 0.119 mmol) in anhydrous methanol (5 ml) and AcOH (0.500 ml) was stirred at room temperature for 10 min, then NaCNBH₃ (7.51 mg, 0.119 mmol) was added in one portion. The reaction mixture was stirred at room temperature for 16h at rt. Reaction mixture was diluted with DCM (5 ml) and washed with water (5 ml). Organic layer was washed with K₂CO₃ saturated solution (2x10 ml), dried over Na₂SO₄, filtered and concentrated under reduced pressure. The crude was purified by column chromatography eluting with H₂O/MeCN/HCO₂H, from 95:5:0.1 to 5:95:0.1 to provide the title compound (22 mg, 33%). Deblocking of formic acid salt was accomplished by SCX, recovering pure product as free base (13 mg, 100%).

| **Example No.** | **Analytical Data** |
|---|---|
| 3 | ¹H NMR (DMSO-d6, 400MHz): d = 10.31 (s, 1H), 9.13 (s, 1H), 8.84 (s, 2H), 8.15 (s, 2H), 7.99 (s, 1H), 7.33 (s, 1H), 7.30 (br d, *J*=7.9 Hz, 1H), 7.16-7.26 (m, 2H), 4.45 (s, 2H), 3.53 (s, 2H), 3.37 (br t, *J*=8.3 Hz, 2H), 3.00 (br t, *J*=8.3 Hz, 2H), 2.25-2.47 (m, 8H), 2.18 ppm (s, 3H) |
| | LC-MS (ESI): (Method 2) t*_{R}* = 0.68 min; m/z (M+1)= 511 |

The compounds reported in the following table were prepared via reductive amination as described for Example 3, step 1-3, applying the corresponding, commercially available or previously synthesized aldehyde in step 3. Modifications of reductive agent, solvent or chromatographic purification conditions were reported in the table.

| **Example No** | **Structure** | **Reagents Amount** | **Product Amount (yield)** | **Purification method** | **Data** |
|---|---|---|---|---|---|
| 18 | 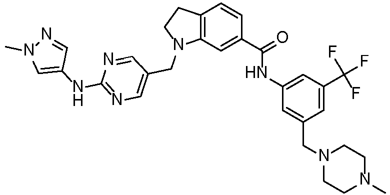 | Titanium(IV) isopropoxide, STAB, DCM Intermediate 4: 87 mg (1.2 eq) Intermediate 8: 150 mg | 100mg (46%) | FCC | 1H NMR (300 MHz, Methanol-d4) δ 8.42 (s, 2H), 8.07 (s, 1H), 7.94 (d, J = 0.8 Hz, 1H), 7.91 (s, 1H), 7.54 (d, J = 0.8 Hz, 1H), 7.41 (s, 1H), 7.28 (dd, J = 7.5, 1.6 Hz, 1H), 7.24 - 7.16 (m, 2H), 4.26 (s, 2H), 3.85 (s, 3H), 3.61 (s, 2H), 3.36 (t, J = 8.3 Hz, 2H), 3.01 (t, J = 8.3 Hz, 2H), 2.53 (s, 8H), 2.29 (s, 3H). |
| | | | | | LC-MS (ESI): (Method 4) t*_{R}* = 1.88 min; m/z [M+H] = 606.22 |
| 19 | 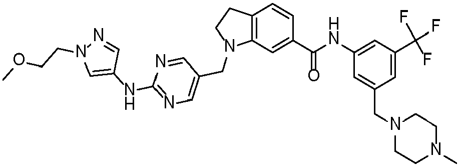 | Titanium(IV) isopropoxide, STAB, DCM Intermediate 21: 87 mg (1.2 eq) Intermediate 8: 150 mg | 20mg (9%) | FCC | ¹H NMR (300 MHz, Methanol-d4) δ 8.43 (s, 2H), 8.06 (s, 1H), 7.99 (d, J = 0.8 Hz, 1H), 7.92 (s, 1H), 7.59 (d, J = 0.8 Hz, 1H), 7.42 (s, 1H), 7.28 (dd, J = 7.5, 1.6 Hz, 1H), 7.20 (d, J = 8.0 Hz, 2H), 4.30 - 4.20 (m, 4H), 3.72 (t, J = 5.2 Hz, 2H), 3.62 (s, 2H), 3.37 (t, J = 8.3 Hz, 14H), 3.02 (t, J = 8.3 Hz, 2H), 2.56 (s, 8H), 2.33 (s, 3H). |
| | | | | | LC-MS (ESI): (Method 4) t*_{R}* = 1.92 min; m/z [M+H] = 650.32 |
| | 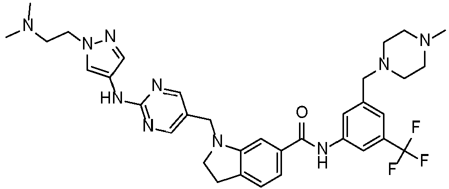 | Titanium(IV) isopropoxide, STAB, DCM | | | ¹H NMR (300 MHz, Methanol-d4) δ 8.43 (s, 2H), 8.07 (s, 1H), 8.03 (s, 1H), 7.91 (s, 1H), 7.58 (s, 1H), 7.41 (s, 1H), 7.28 (dd, J = 7.5, 1.6 Hz, 1H), 7.23 - 7.17 (m, 2H), 4.27 (s, 2H), 4.23 (t, J = 6.9 Hz, 2H), 3.61 (s, 2H), 3.37 (t, J = 8.3 Hz, 2H), 3.01 (t, J = 8.3 Hz, 2H), 2.80 (t, J = 6.9 Hz, 2H), 2.53 (s, 8H), 2.29 (s, 3H), 2.28 (s, 6H). |
| 20 | | Intermediate 17: 349 mg (1.34 eq) | 41mg (47%) | Prep HPLC | |
| | | Intermediate 8: 561 mg | | | |
| | | | | | LC-MS (ESI): (Method 4) t*_{R}* = 2.34 min; m/z [M+H] = 663.31 |

### Example 4: Preparation of N-(1-((2-((1-methyl-1H-pyrazol-4-yl)amino)pyrimidin-5-yl)methyl)indolin-6-yl)-3-((4-methylpiperazin-1-yl)methyl)-5-(trifluoromethyl) benzamide

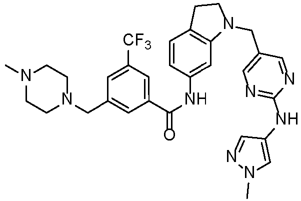

### Example 4

### Step 1; tert-butyl 6-nitroindoline-1-carboxylate - Intermediate 9

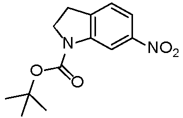

BOC-Anhydride (26.6 g, 122 mmol), 6-nitroindoline (20 g, 122 mmol), DMAP (1.5 g, 12.18 mmol), TEA (51 ml, 365 mmol), DCM (244 ml) were put into the flask. The reaction mixture was stirred at rt overnight. Solvents were removed under vacuum. Extraction with DCM was done. Solvents were removed again. Crude product was purified by FCC using as eluent hexane/ethyl acetate 9:1 to give the title compound (29 g, 90%).

H NMR (300 MHz, DMSO-d6) δ 8.42 (s, 1H), 7.84 (dd, J = 8.2, 2.3 Hz, 1H), 7.49 - 7.40 (m, 1H), 4.01 (dd, J = 9.2, 8.2 Hz, 2H), 3.19 (t, J = 8.7 Hz, 2H), 1.54 (s, 9H).

### Step 2; tert-butyl 6-aminoindoline-1-carboxylate - Intermediate 10

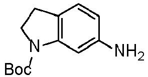

Into the 3-neck round button was charged **Intermediate 9** (29.13 g, 110 mmol). Air was evacuated using water pump and purged with Argon - step was repeated 3 times. After that Palladium on Carbon 10 wt. % (2.91 g, 27.3 mmol) and Ethyl acetate (551 ml) was added. The reaction mixture was purged with Argon and removed using water pump, next purged with Hydrogen. The mixture was stirred in hydrogen atmosphere at rt overnight. The reaction mixture was filtered through celite pad and concentrated to give the title compound (27 g, 100%).

¹H NMR (300 MHz, DMSO-d6) δ 7.05 (s, 1H), 6.80 (d, J = 7.9 Hz, 1H), 6.13 (dd, J = 7.9, 2.1 Hz, 1H), 4.92 (d, J = 6.6 Hz, 2H), 3.82 (dd, J = 9.2, 7.9 Hz, 2H), 2.86 (t, J = 8.5 Hz, 2H), 1.50 (s, 9H).

### Step 3; tert-butyl 6-(3-((4-methylpiperazin-1-yl)methyl)-5-(trifluoromethyl)benzamido)indoline-1-carboxylate - Intermediate 11

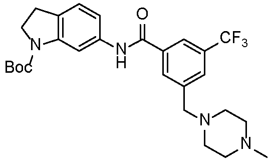

Intermediate 10 (6.45 g, 21.34 mmol) was placed in to the flask then HATU (16.23 g, 42.7 mmol), DIPEA (14.91 ml, 85 mmol), DMF (53.4 ml) and Dichloromethane (160 ml) were added. The reaction mixture was stirred at rt for 30 min. After the time tert-butyl 6-aminoindoline-1-carboxylate (5 g, 21.34 mmol) was added. The stirring was continued overnight. Extraction with water and DCM was done. Organic phase was removed under reduce pressure. Crude product was purified by FCC eluting with hexane/ethyl acetate 9:1 to provide the title compound (10.4 g, 92%).

¹H NMR (300 MHz, DMSO-d6) δ 10.40 (s, 1H), 9.32 (s, 1H), 8.24 (d, J = 17.0 Hz, 2H), 8.15 (d, J = 1.9 Hz, 1H), 7.90 (s, 1H), 7.18 (d, J = 8.0 Hz, 1H), 3.94 (t, J = 8.5 Hz, 2H), 3.78 (s, 2H), 3.05 (t, J = 8.6 Hz, 2H), 2.70 (s, 3H), 2.40 (s, 8H), 1.52 (s, 9H).

### Step 4; N-(indolin-6-yl)-3-((4-methylpiperazin-1-yl)methyl)-5-(trifluoromethyl)benzamide - Intermediate 12

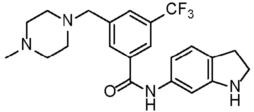

Intermediate 11 (1.938 g, 3.74 mmol) was dissolved in DCM (37.4 ml) and TFA (2.59 ml, 33.6 mmol) was added. The reaction mixture was stirred at rt overnight. The mixture was washed with sat NaHCO₃ and brine (10mL). The organic phase was separated, dried over Na₂SO₄, filtered and concentrated to dryness. Crude material was purified via column chromatography and eluting system DCM/MeOH, 95:5. The pure fractions were washed with water, brine and 5% citric acid and finally the organic phase was dried over Na₂SO₄ and evaporated under vacuum to give the title compound (770 mg, 48%).

¹H NMR (300 MHz, DMSO-d6) δ 10.16 (s, 1H), 8.14 (d, J = 6.8 Hz, 2H), 7.83 (s, 1H), 7.04 (d, J = 1.9 Hz, 1H), 6.98 (d, J = 7.9 Hz, 1H), 6.92 - 6.82 (m, 1H), 5.61 (s, 1H), 3.63 (s, 2H), 3.48 - 3.39 (m, 2H), 2.88 (t, J = 8.4 Hz, 2H), 2.37 (d, J = 22.8 Hz, 8H), 2.16 (s, 3H).

### Step 5; N-(1-((2-((1-methyl-1H-pyrazol-4-yl)amino)pyrimidin-5-yl)methyl)indolin-6-yl)-3-((4-methylpiperazin-1-yl)methyl)-5-(trifluoromethyl) benzamide (Example 4)

Intermediate 12 (0.065 g, 0.155 mmol) was dissolved in anhydrous DCM (2.4 ml) then 2-((1-methyl-1H-pyrazol-4-yl)amino)pyrimidine-5-carbaldehyde (0.032 g, 0.155 mmol) was added followed by AcOH (17.78 µl, 0.311 mmol) and magnesium sulfate (37.4 mg, 0.311 mmol). Reaction mixture was stirred 30 min at rt. Next STAB (65.8 mg, 0.311 mmol) was added and reaction mixture stirred at rt overnight. The mixture was quenched with addition of sat. NaHCO₃. The phases were separated and the water layer was washed with DCM (x2). Collected organic phases were dried over Na₂SO₄ and solvent was removed under vacuum. Crude material was purified by FCC eluting with DCM/MeOH 4:1 and by preparative HPLC eluting with ACN, H₂O + 0.05% NH₃ to give the title compound (56 mg, 21 %).

| **Example No.** | **Analytical Data** |
|---|---|
| 4 | ¹H NMR (300 MHz, DMSO-d6) δ 10.27 (s, 1H), 9.43 (s, 1H), 8.42 (s, 2H), 8.16 (d, J = 7.2 Hz, 2H), 7.88 (s, 1H), 7.84 (s, 1H), 7.45 (d, J = 0.8 Hz, 1H), 7.14 (s, 1H), 7.01 (s, 2H), 4.16 (s, 2H), 3.78 (s, 3H), 3.63 (s, 2H), 2.86 (t, J = 8.3 Hz, 2H), 2.37 (d, J = 23.7 Hz, 8H), 2.15 (s, 3H). |
| | LC-MS (ESI): (Method 1) t*_{R}=* 1.90 min; m/z (M+1)= 606.30 |

The following compounds were prepared via reductive amination as described for **Example 4,** step 1, applying the corresponding, commercially available aldehyde in step 1

| **Example No** | **Structure** | **Reagents Amount** | **Product Amount (yield)** | **Method purification** | **Data** |
|---|---|---|---|---|---|
| 5 | 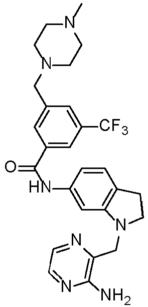 | 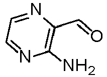 (29 mg, 0.24 mmol) Intermediate 12 100 g | 0.023 g (Y=18%) | FCC and prep HPLC | ¹H NMR (300 MHz, DMSO-d6) δ 10.23 (s, 1H), 8.14 (d, J = 7.9 Hz, 2H), 7.88 (d, J = 2.7 Hz, 1H), 7.82 (s, 1H), 7.71 (d, J = 2.7 Hz, 1H), 7.03 (d, J = 1.1 Hz, 2H), 6.97 (s, 1H), 6.22 (s, 2H), 4.26 (s, 2H), 3.62 (s, 2H), 3.38 (d, J = 8.5 Hz, 2H), 2.90 (t, J = 8.2 Hz, 2H), 2.37 (d, J = 22.6 Hz, 8H), 2.15 (s, 3H). LC-MS (ESI): (Method 1) t*_{R} =* 1.70 min; m/z (M+1)= 526.25 |
| 21 | 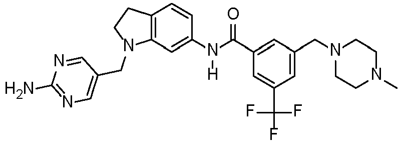 | 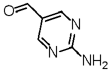 (14.7 mg, 0.12 mmol) Intermediate 12, 50 mg | 26mg (Y=43%) | FCC | ¹H NMR (400 MHz, DMSO) δ 10.27 (s, 1H), 8.26 (s, 2H), 8.20 - 8.15 (m, 2H), 7.85 (s, 1H), 7.13 (s, 1H), 7.02 - 7.01 (m, 2H), 6.59 (s, 2H), 4.09 (s, 2H), 3.65 (s, 2H), 3.28 (t, J=8.3 Hz, 2H), 2.86 (t, J=8.2 Hz, 2H), 2.36 - 2.34 (m, 8H), 2.17 (s, 3H) LC-MS (ESI): (Method 3) t*_{R}* = 3.11 min; m/z [M+H]⁺= 526.2 |

### Example 6: Preparation of N-methyl-4-((6-(2-(3-(4-methyl-1H-imidazol-1-yl)-5-(trifluoromethyl)phenyl)-2-oxoethyl)indolin-1-yl)methyl)picolinamide

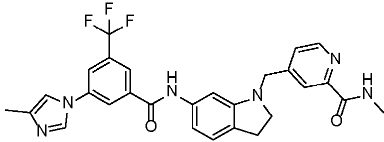

### Example 6

### Step 1; tert-butyl 6-(3-(4-methyl-1H-imidazol-1-yl)-5-(trifluoromethyl) benzamido)indoline-1-carboxylate - Intermediate 13

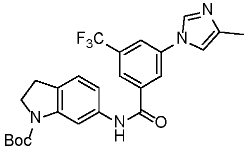

3-(4-methyl-1H-imidazol-1-yl)-5-(trifluoromethyl)benzoic acid (1.15 g, 4.27 mmol), tert-butyl 6-aminoindoline-1-carboxylate(1.0 g, 4.27 mmol), HATU (4.87 g, 12.80 mmol), DIPEA (4.47 ml, 25.6 mmol) were put in to the flask then DMF (0.907 ml) and DCM (2.721 ml) were added. The reaction mixture was stirred at rt overnight. Extraction with water and DCM was done. Solvents were dried over Na₂SO₄ and removed under reduce pressure. Crude material was purified by FCC eluting with Hexane/ethyl acetate 1:1 to give the title compound (2.2 g, 90%).

¹H NMR (300 MHz, DMSO-d6) δ 10.52 (s, 1H), 9.55 (s, 1H), 8.58 (s, 1H), 8.43 (d, J = 8.1 Hz, 2H), 8.15 (s, 2H), 7.21 (d, J = 8.0 Hz, 1H), 4.01 - 3.90 (m, 2H), 3.06 (t, J = 8.5 Hz, 2H), 2.36 (d, J = 1.2 Hz, 3H), 1.52 (s, 9H).

### Step 2; N-(indolin-6-yl)-3-(4-methyl-1H-imidazol-1-yl)-5-(trifluoromethyl) benzamide - Intermediate 14

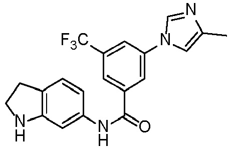

Intermediate 13 (1.87 g, 3.84 mmol) was dissolved in DCM (38.4 ml). Then TFA (2.67 ml, 34.6 mmol) was added. Reaction mixture was stirred at rt overnight. After washing with NaHCO₃, DCM was dried over Na₂SO₄. Solvents were removed under reduce pressure. Crude product was purified by FCC eluting with DCM/MeOH 95:5. After that, extraction with water, brine and 5% citric acid was done to give the title compound (1.09 g, 53%).

¹H NMR (300 MHz, DMSO-d6) δ 10.21 (s, 1H), 8.40 (d, J = 1.5 Hz, 2H), 8.22 (s, 1H), 8.13 (s, 1H), 7.72 (d, J = 1.4 Hz, 1H), 7.05 (d, J = 1.9 Hz, 1H), 7.00 (d, J = 7.8 Hz, 1H), 6.89 (d, J = 8.0 Hz, 1H), 5.64 (s, 1H), 3.44 (d, J = 8.4 Hz, 2H), 2.89 (t, J = 8.4 Hz, 2H), 2.20 (s, 3H).

### Step 3; N-methyl-4-((6-(2-(3-(4-methyl-1H-imidazol-1-yl)-5-(trifluoro methyl)phenyl) -2-oxoethyl)indolin-1-yl)methyl)picolinamide (Example 6)

To a flask were put Intermediate 14 (100 mg, 0.259 mmol), 4-formyl-N-methylpicolinamide (42 mg, 0.259 mmol), then acetic acid (30 µl, 0.518 mmol) and DCM (2.4 ml) was added. The reaction mixture was stirred at rt for 1/2h. After the time NaBH₃CN (62.8 mg, 0.388 mmol) was added and mixture was stirred overnight. The mixture was quenched with addition of sat. NaHCO₃. The phases were separated and the water layer was washed with DCM (x2). The all combined organic phases were dried over Na₂SO₄, filtered and concentrated under reduce pressure. Crude material was purified by column chromatography eluting with DCM/MeOH 95:05 and by preparative HPLC eluting with ACN, H₂O + 0.05% NH₃ to afford the title compound (40 mg, 28%).

| **Example No.** | **Analytical Data** |
|---|---|
| 6 | ¹H NMR (300 MHz, DMSO-d6) δ 10.28 (s, 1H), 8.79 (d, J = 4.9 Hz, 1H), 8.60 (dd, J = 4.9, 0.8 Hz, 1H), 8.39 (d, J = 1.5 Hz, 2H), 8.21 (s, 1H), 8.11 (s, 1H), 8.02 (d, J = 1.6 Hz, 1H), 7.70 (t, J = 1.3 Hz, 1H), 7.57 (dd, J = 5.0, 1.7 Hz, 1H), 7.12 - 7.01 (m, 2H), 6.92 (s, 1H), 4.42 (s, 2H), 3.43 (t, J = 8.3 Hz, 2H), 2.98 (t, J = 8.3 Hz, 2H), 2.82 (d, J = 4.8 Hz, 3H), 2.19 (d, J = 1.0 Hz, 3H). |
| | LC-MS (ESI): (Method 1) t*_{R}* = 2.08 min; m/z (M+1)= 535.13 |

### Example 7: Preparation of 1-((5-(4-methyl-3-oxopiperazin-1-yl)pyridin-3-yl)methyl)-N-(3-(trifluoromethyl)phenyl)indoline-6-carboxamide

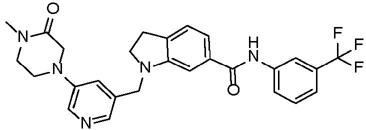

### Example 7

### Step 1; methyl 1-((5-bromopyridin-3-yl)methyl)indoline-6-carboxylate (Intermediate 23)

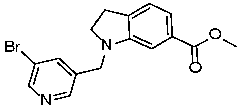

Following the procedure as per Example 4, step 5, starting from methyl indolin-1-ium-6-carboxylate hydrochloride (1 g, 4.68 mmol) and 5-bromo-3-pyridinecarboxaldehyde (871 mg, 4.68 mmol) title compound was obtained (1.54 g, 87 %)^{.}

¹HNMR (400 MHz, DMSO) δ 8.88 - 8.79 (m, 2H), 8.68 - 8.68 (m, 1H), 8.36 - 8.35 (m, 1H), 7.76 - 7.70 (m, 1H), 7.46 - 7.42 (m, 1H), 4.09 (t, J=8.2 Hz, 2H), 3.88 - 3.84 (m, 3H), 3.18 (t, J=8.3 Hz, 2H)

### Step 2; 1-((5-bromopyridin-3-yl)methyl)indoline-6-carboxylic acid (Intermediate 24)

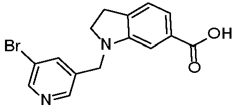

To a solution of Intermediate 23 (1 g, 2.88 mmol) in MeOH (15 mL) was added lithium hydroxide monohydrate (363 mg, 8.64 mmol) in water (15 mL) and the resulting mixture was stirred at rt for 3 days, then 40°C for 2h. The reaction mixture was cooled to room temperature, concentrated and acidified with 1M HCl (aq). The resulting precipitate was filtered, washed with water and dried to give title compound (890 mg, 93 %).

¹H NMR (400 MHz, DMSO) δ 12.62 (s, 1H), 8.63 (d, J=2.0 Hz, 1H), 8.57 (s, 1H), 8.03 (s, 1H), 7.27 (d, J=7.5 Hz, 1H), 7.15 (d, J=7.5 Hz, 1H), 7.08 (s, 1H), 4.38 (s, 2H), 3.38 (t, J=8.2 Hz, 2H), 2.98 (t, J=8.3 Hz, 2H).

### Step 3; 1-((5-bromopyridin-3-yl)methyl)-N-(3-(trifluoromethyl)phenyl) indoline-6-carboxamide (Intermediate 25)

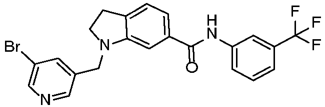

Following the procedure as per Intermediate 11, starting from Intermediate 24 (500 mg, 1.50 mmol) and 3-(trifluoromethyl)aniline (0.20 mL, 1.58 mmol) title compound was obtained (373 mg, 52%).

¹H NMR (400 MHz, DMSO) δ 10.33 (s, 1H), 8.65 (d, J=2.3 Hz, 1H), 8.60 (d, J=1.8 Hz, 1H), 8.22 (s, 1H), 8.06 (t, J=2.1 Hz, 1H), 8.03 (d, J=9.3 Hz, 1H), 7.58 (t, J=8.0 Hz, 1H), 7.44 - 7.41 (m, 1H), 7.29 (q, J=3.0 Hz, 1H), 7.21 (d, J=7.6 Hz, 1H), 7.16 (d, J=1.2 Hz, 1H), 4.43 (s, 2H), 3.38 (t, J=8.2 Hz, 2H), 3.01 (t, J=8.3 Hz, 2H)

### Step 4; 1-((5-(4-methyl-3-oxopiperazin-1-yl)pyridin-3-yl)methyl)-N-(3-(trifluoromethyl)phenyl)indoline-6-carboxamide (Example 7)

A mixture of Intermediate 25 (70 mg, 0.147 mmol), 1-methylpiperazin-2-one (18 mg, 0.162 mmol) and cesium carbonate (96 mg, 0.294 mmol) in anhydrous 1,4-dioxane (2.00 mL) was degassed and RuPhos Pd G3 (12 mg, 0.0147 mmol) was added. The reaction mixture was heated and stirred at 80°C under argon for 18h. The reaction mixture was cooled, filtered through a pad of celite and concentrated. The crude compound was purified by preparative HPLC (Sunfire C18 19x150mm, 10um 20-80% ACN / H2O (10mM NH₄CO₃), 20 ml/min, RT) to give the title compound (44 mg, 57 %) as an off-white solid.

LC-MS (ESI): (Method 3) t*_{R}* = 3.58 min; m/z [M+H]⁺= 510.5

¹H NMR (400 MHz, DMSO) δ 10.34 (s, 1H), 8.26 - 8.24 (m, 2H), 8.06 (d, J=1.6 Hz, 1H), 8.04 (s, 1H), 7.60 (t, J=8.0 Hz, 1H), 7.45 (d, J=7.8 Hz, 1H), 7.36 (t, J=2.2 Hz, 1H), 7.29 (q, J=3.0 Hz, 1H), 7.23 - 7.20 (m, 2H), 4.36 (s, 2H), 3.85 (s, 2H), 3.57 (dd, J=4.5, 6.3 Hz, 2H), 3.44 (dd, J=4.3, 6.4 Hz, 2H), 3.38 (t, J=8.5 Hz, 2H), 3.00 (t, J=8.3 Hz, 2H), 2.91 (s, 3H).

### Example 8: Preparation of 1-((2-Aminopyrimidin-5-yl)methyl)-N-(4-((4-methylpiperazin-1-yl)methyl)-3-(trifluoromethyl)phenyl)indoline-6-carboxamide

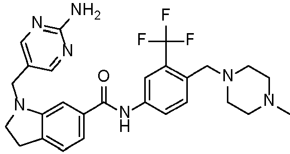

### Example 8

### Step 1; tert-butyl 6-((4-((4-methylpiperazin-1-yl)methyl)-3-trifluoromethyl) phenyl)carbamoyl)indoline-1-carboxamide (Intermediate 26)

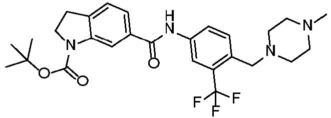

To a suspension of 1-tert-butoxycarbonylindoline-6-carboxylic acid (1.00 g, 3.80 mmol) and 4-[(4-methylpiperazin-1-yl)methyl]-3-(trifluoromethyl)aniline (1.25 g, 4.56 mmol) in DMF (10 mL) was added 1-methylimidazole (1.1 mL, 13.3 mmol) and chloro-N,N,N',N'-tetramethylformamidinium hexafluorophosphate (1.60 g, 5.70 mmol). The reaction mixture was stirred at RT for 24h. The mixture was partitioned between brine and ethyl acetate. The organic phase was dried (Na₂SO₄) and concentrated. The residue was purified by FCC (0-10% 2M NH₃/MeOH in DCM) to give title compound (2.22 g, 100%)

LCMS (Method 5) t*_{R}* = 1.72 min, m/z [M+H] = 519

### Step 2; N-((4-((4-methylpiperazin-1-yl)methyl)-3-(trifluoromethyl)phenyl) carbamoyl)indoline-6-carboxamide (Intermediate 27)

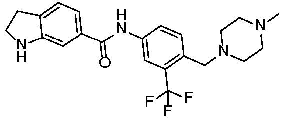

Following the procedure as per Intermediate 12, starting from Intermediate 26 (2.22 g, 4.28 mmol), in DCM (15 mL) title compound was obtained (1.47 g, 82 %).

¹H NMR (400 MHz, DMSO) δ 10.28 - 10.26 (m, 1H), 8.21 - 8.19 (m, 1H), 8.01 (dd, J=1.9, 8.5 Hz, 1H), 7.68 - 7.65 (m, 1H), 7.16 - 7.15 (m, 2H), 7.03 (s, 1H), 5.75 (s, 1H), 3.55 (s, 2H), 3.48 (dt, J=1.4, 8.6 Hz, 2H), 3.00 - 2.94 (m, 2H), 2.38 - 2.33 (m, 8H), 2.16 - 2.15 (m, 3H)

### Step 3; 1-((2-Aminopyrimidin-5-yl)methy)1-N-(4-((4-methylpiperazin-1-yl) methyl)-3-(trifluoromethyl)phenyl)indoline-6-carboxamide (Example 8)

Following procedure as per Example 4, step 3, starting from 2-aminopyrimidine-5-carboxaldehyde (18 mg, 0.149 mmol) and Intermediate 27 (50 mg, 0.119 mmol), after purification by HPLC (Xbridge Phenyl 19x150mm, 10um 40-100% MeOH / H₂O (10mM NH₄CO₃), 20 ml/min, RT) the title compound was obtained (50 mg, 79%).

LC-MS (ESI): (Method 3) t*_{R}* = 3.07 min; m/z [M+H]⁺= 526.2

¹H NMR (400 MHz, DMSO) δ 10.30 (s, 1H), 8.26 (s, 2H), 8.21 (d, J=2.4 Hz, 1H), 8.04 (dd, J=2.1, 8.6 Hz, 1H), 7.70 (d, J=8.7 Hz, 1H), 7.29 - 7.17 (m, 3H), 6.61 (s, 2H), 4.18 (s, 2H), 3.58 (s, 2H), 3.29 (t, J=7.6 Hz, 2H), 2.99 - 2.93 (m, 2H), 2.40 - 2.34 (m, 8H), 2.18 - 2.17 (m, 3H)

The compounds reported in the following table were prepared as described for Example 8, step 3, applying the corresponding, commercially available aldehyde in step 3.

| **Example No** | **Structure** | **Amount Reagents** | **Product Amount (yield)** | **Method purification** | **Data** |
|---|---|---|---|---|---|
| 9 | 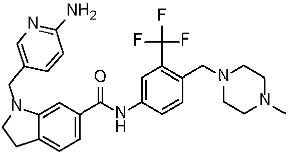 | 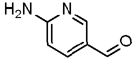 (15 mg, 0.119 mmol) | 29 mg, (57%) | Prep HPLC | ¹H NMR (400 MHz, DMSO) δ 10.28 (s, 1H), 8.21 (d, J=2.1 Hz, 1H), 8.04 (dd, J=2.1, 8.5 Hz, 1H), 7.92 (d, J=1.9 Hz, 1H), 7.70 (d, J=8.4 Hz, 1H), 7.37 (dd, J=2.4, 8.4 Hz, 1H), 7.25 (dd, J=1.4, 7.5 Hz, 1H), 7.21 (d, J=1.2 Hz, 1H), 7.18 (d, J=7.7 Hz, 1H), 6.44 (dd, J=0.7, 8.4 Hz, 1H), 5.87 (s, 2H), 4.18 (s, 2H), 3.57 (s, 2H), 3.28 (t, J=8.5 Hz, 2H), 2.98 - 2.91 (m, 2H), 2.41 - 2.34 (m, 8H), 2.18 - 2.17 (m, 3H) |
| | | Intermediate 27 40 mg | | | |
| | | | | | LC-MS (ESI): method Jo 7 t*_{R}* = 2.67 min; m/z [M+H]+ = 525.4 |
| 10 | 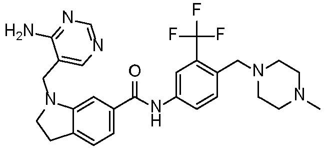 | 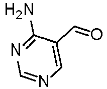 (15 mg, 0.119 mmol) | 38 mg, (76%) | Prep HPLC | ¹H NMR (400 MHz, DMSO) δ 10.30 (s, 1H), 8.35 (s, 1H), 8.20 (d, J=2.3 Hz, 1H), 8.09 (s, 1H), 8.03 (dd, J=1.9, 8.8 Hz, 1H), 7.72 - 7.68 (m, 1H), 7.33 (dd, J=1.4, 7.5 Hz, 1H), 7.23 (d, J=7.6 Hz, 1H), 7.19 (d, J=1.2 Hz, 1H), 6.78 (br s, 2H), 4.17 (s, 2H), 3.57 (s, 2H), 3.31 (t, J=7.4 Hz, 2H), 3.00 (t, J=8.3 Hz, 2H), 2.40 - 2.31 (m, 8H), 2.17 (s, 3H) |
| | | Intermediate 27 40 mg | | | |
| | | | | | LC-MS (ESI): (Method 3) |
| | | | | | t*_{R}* = 2.53 min; m/z [M+H]⁺= 526.2 |
| 11 | 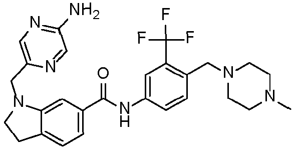 | 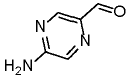 (19 mg, 0.119 mmol) | 14 mg, (27%) | SFC Waters Torus 2 PIC 10x250mm, 5um 20-30% MeOH (0.1% DEA) / CO₂, 15ml/min, 120bar, 40C, DAD | ¹H NMR (400 MHz, DMSO) δ 10.28 (s, 1H), 8.21 (d, J=2.1 Hz, 1H), 8.04 (dd, J=2.1, 8.5 Hz, 1H), 7.95 (d, J=1.4 Hz, 1H), 7.87 (d, J=1.4 Hz, 1H), 7.70 (d, J=8.5 Hz, 1H), 7.24 (dd, J=1.4, 7.6 Hz, 1H), 7.18 (s, 1H), 7.17 (d, J=1.5 Hz, 1H), 6.36 (s, 2H), 4.29 (s, 2H), 3.57 (s, 2H), 3.42 (t, J=8.5 Hz, 2H), 2.97 (t, J=8.3 Hz, 2H), 2.41 - 2.33 (m, 8H), 2.17 (s, 3H) |
| | | Intermediate 27 40 mg | | | |
| | | | | | LC-MS (ESI): (Method 3) |
| | | | | | t*_{R}* = 3.23 min; m/z [M+H]⁺= 526.2 |
| 12 | 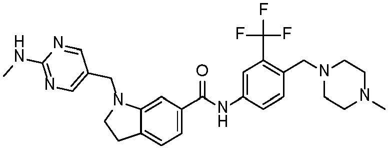 | 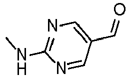 (20 mg, 0.149 mmol) | 43 mg, (67%) | Prep HPLC | ¹H NMR (400 MHz, DMSO) δ 10.30 (s, 1H), 8.31 (s, 2H), 8.21 (d, J=2.1 Hz, 1H), 8.04 (dd, J=2.0, 8.5 Hz, 1H), 7.70 (d, J=8.4 Hz, 1H), 7.28 - 7.24 (m, 2H), 7.19 (d, J=7.5 Hz, 1H), 7.08 (q, J=4.6 Hz, 1H), 4.20 (s, 2H), 3.58 (s, 2H), 3.30 (dd, J=8.3, 8.3 Hz, 2H), 2.95 (dd, J=8.3, 8.3 Hz, 2H), 2.80 (d, J=4.8 Hz, 3H), 2.38 - 2.34 (m, 6H), 2.18 (s, 3H). 2H not observed and assumed to be overlapping with solvent peaks |
| | | Intermediate 27 50 mg | | | |
| | | | | | LC-MS (ESI): (Method 3) |
| | | | | | t*_{R}* = 3.25 min; m/z [M+H]⁺= 540.4 |
| 13 | 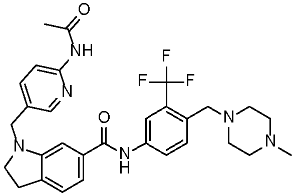 | 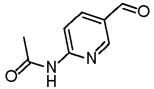 (25 mg, 0.149 mmol) | 44 mg, (65%) | Prep HPLC | ¹H NMR (400 MHz, DMSO) δ 10.50 (s, 1H), 10.29 (s, 1H), 8.33 (d, J=2.0 Hz, 1H), 8.20 (d, J=2.1 Hz, 1H), 8.10 - 8.01 (m, 2H), 7.78 (dd, J=2.3, 8.6 Hz, 1H), 7.70 (d, J=8.5 Hz, 1H), 7.30 - 7.26 (m, 1H), 7.22 - 7.19 (m, 2H), 4.35 (s, 2H), 3.57 (s, 2H), 2.98 (dd, J=8.4, 8.4 Hz, 2H), 2.46 - 2.28 (m, 8H), 2.17 (s, 3H), 2.10 - 2.09 (m, 3H). 2H not observed and assumed to be overlapping with solvent peaks. |
| | | Intermediate 27 50 mg | | | |
| | | | | | LC-MS (ESI): (Method 3) |
| | | | | | t*_{R}* = 3.29 min; m/z [M+H]⁺= 567.4 |
| 14 | 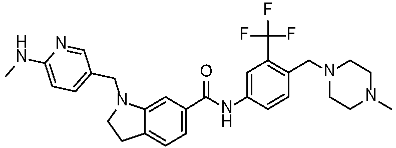 | 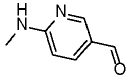 (24 mg, 0.15 mmol) | 40 mg, (52%) | Prep HPLC | ¹H NMR (400 MHz, DMSO) δ 10.29 (s, 1H), 8.21 (d, J=2.1 Hz, 1H), 8.04 (dd, J =2.1, 8.5 Hz, 1H), 7.99 (d, J=2.1 Hz, 1H), 7.70 (d, J=8.5 Hz, 1H), 7.38 (dd, J=2.4, 8.5 Hz, 1H), 7.27 - 7.22 (m, 2H), 7.18 (d, J=7.5 Hz, 1H), 6.45 - 6.42 (m, 2H), 4.19 (s, 2H), 3.57 (s, 2H), 3.28 (t, J=8.5 Hz, 2H), 2.94 (t, J=8.4 Hz, 2H), 2.76 (d, J=4.9 Hz, 3H), 2.40 - 2.34 (m, 6H), 2.17 (s, 3H). 2 missing pyrazine protons under solvent peaks. |
| | | Intermediate 27 50 mg | | | |
| | | | | | LC-MS (ESI): (Method 3) |
| | | | | | t*_{R}* = 2.64 min; m/z [M+H]⁺= 539.2 |
| 15 | 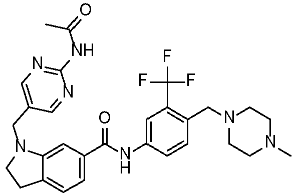 | 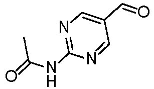 Intermediate 22 (30 mg, 0.18 mmol) | 36 mg, (44%) | Prep HPLC | ¹H NMR (400 MHz, DMSO) δ 10.57 (s, 1H), 10.31 (s, 1H), 8.67 (s, 2H), 8.21 (d, J=2.1 Hz, 1H), 8.04 (dd, J=2.1, 8.5 Hz, 1H), 7.70 (d, J=8.5 Hz, 1H), 7.31 - 7.28 (m, 1H), 7.25 - 7.20 (m, 2H), 4.37 (s, 2H), 3.57 (s, 2H), 3.40 - 3.35 (m, 2H), 2.99 (t, J=8.3 Hz, 2H), 2.38 - 2.33 (m, 6H), 2.19 (s, 3H), 2.17 (s, 3H). 2 missing pyrazine protons under solvent peaks. |
| | | Intermediate 27 60 mg | | | |
| | | | | | LC-MS (ESI): (Method 3) |
| | | | | | t*_{R}* = 3.06 min; m/z [M+H]⁺= 568.2 |

### Example 16: Preparation of 1-((2-((1-(2-methoxyethyl)-1H-pyrazol-4-yl)amino)pyrimidin-5-yl)methyl)-N-(3-(trifluoromethyl)phenyl)indoline-6-carboxamide

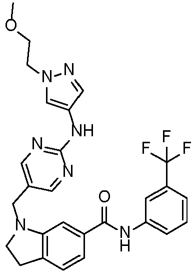

### Example 16

### Step 1; tert-butyl 3-methyl-6-((3-(trifluoromethyl)phenyl)carbamoyl)indoline-1-carboxylate (Intermediate 28)

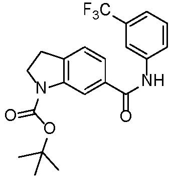

3-(trifluoromethyl)aniline (2.027 ml, 16.23 mmol) was dissolved in dry THF (27.0 ml) and this solution was cooled down to -78°C. Next n-BuLi (5.41 ml, 13.52 mmol) was added and the mixture was stirred for 30 min, followed by addition of 1-(tert-butyl) 6-methyl indoline-1,6-dicarboxylate (1.5g, 5.41 mmol) in anh. THF (27.0 ml). The mixture was stirred in dry ice bath overnight. The reaction mixture was quenched by addition of water and the solution was extracted with AcOEt. All organic layer was combined, dried over Na₂SO₄, filtered and concentrated.

The crude material was purfied by FCC (eluting system: from Hex 100% to Hex/AcOEt 1/1). Then was dissolved in AcOEt and extracted with 0.5M HCl to afford title compound (1.70 g, 4.2 mmol, 77%)

¹H NMR (300 MHz, DMSO-d6) δ 10.52 (s, 1H), 8.23 (d, J = 2.0 Hz, 2H), 8.09 - 8.00 (m, 1H), 7.63 - 7.52 (m, 2H), 7.44 (ddd, J = 7.8, 2.0, 1.0 Hz, 1H), 7.35 (d, J = 7.8 Hz, 1H), 3.97 (t, J = 8.7 Hz, 2H), 3.14 (t, J = 8.6 Hz, 2H), 1.53 (s, 9H).

### Step 2; 3-methyl-N-(3-(trifluoromethyl)phenyl)indoline-6-carboxamide (Intermediate 29)

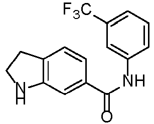

Following the procedure as per Intermediate 12, starting from Intermediate 28 (560 mg, 1.332 mmol) title compound was obtained (375 mg, 86%).

¹H NMR (300 MHz, DMSO-d6) δ 10.33 (s, 1H), 8.25 (d, J = 2.0 Hz, 1H), 8.03 (dt, J = 8.0, 1.3 Hz, 1H), 7.57 (t, J = 8.0 Hz, 1H), 7.41 (ddt, J = 7.8, 1.9, 0.9 Hz, 1H), 7.20 - 7.12 (m, 2H), 7.04 (t, J = 1.0 Hz, 1H), 5.76 (d, J = 2.1 Hz, 1H), 3.48 (td, J = 8.6, 1.9 Hz, 2H), 2.97 (t, J = 8.5 Hz, 2H).

### Step 3; 1-((2-((1-(2-methoxyethyl)-1H-pyrazol-4-yl)amino)pyrimidin-5-yl)methyl)-N-(3-(trifluoromethyl)phenyl)indoline-6-carboxamide (Example 16)

Following the procedure as per Example 4, step 5, starting from Intermediate 29 (148 mg, 0.483 mmol) and 2-((1-(2-methoxyethyl)-1H-pyrazol-4-yl)amino)pyrimidine-5-carbaldehyde (107mg, 0.433 mmol) title compound was obtained (20.4 mg, 0.04 mmol, 8%).

LC-MS (ESI): (Method 1) t*_{R}* = 3.077 min; m/z [M+H]⁺= 538.2

¹H NMR (300 MHz, Methanol-d4) δ 8.43 (s, 2H), 8.15 (s, 1H), 7.99 (d, J = 0.8 Hz, 1H), 7.93 (d, J = 8.3 Hz, 1H), 7.59 (d, J = 0.8 Hz, 1H), 7.54 (t, J = 8.0 Hz, 1H), 7.42 (d, J = 7.8 Hz, 1H), 7.29 (dd, J = 7.5, 1.6 Hz, 1H), 7.23 - 7.18 (m, 2H), 4.28 - 4.23 (m, 4H), 3.72 (t, J = 5.2 Hz, 2H), 3.37 (t, J = 8.3 Hz, 2H), 3.01 (t, J = 8.3 Hz, 2H). Note: OMe Signal not visible because is superposed with solvent signal.

### Example 17: Preparation of 1-[(5-carbamoyl-3-pyridyl)methyl]-N-[4-[(4-methylpiperazin-1-yl)methyl]-3-(trifluoromethyl)phenyl]indoline-6-carboxamide

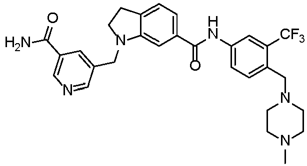

### Example 17

### Step 1; 1-[(5-cyano-3-pyridyl)methyl]-N-[4-[(4-methylpiperazin-1-yl)methyl]-3-(trifluoromethyl)phenyl]indoline-6-carboxamide (Intermediate 30)

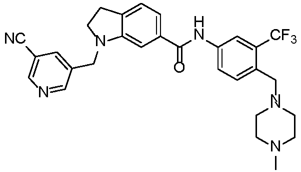

Following the procedure as per Example 4, step 5, starting from Intermediate 27 (100 mg, 0.23 mmol) and 5-formylpyridine-3-carbonitrile (39 mg, 0.3 mmol) the title compound was obtained (128 mg, 100%).

¹H NMR (300 MHz, DMSO) δ 10.30 (s, 1H), 8.99 (d, J=2.0 Hz, 1H), 8.91 (d, J=2.1 Hz, 1H), 8.34 (dd, J=2.1, 2.1 Hz, 1H), 8.20 (d, J=2.3 Hz, 1H), 8.03 (dd, J=2.0, 8.4 Hz, 1H), 7.70 (d, J=8.5 Hz, 1H), 7.30 (dd, J=1.4, 7.5 Hz, 1H), 7.23 (d, J=7.5 Hz, 1H), 7.17 (s, 1H), 4.49 (s, 2H), 3.57 (s, 2H), 3.44 - 3.37 (m, 2H), 3.03 (dd, J=8.3, 8.3 Hz, 2H), 2.50 - 2.34 (m, 8H), 2.18 (s, 3H)

### Step 2; 1-[(5-carbamoyl-3-pyridyl)methyl]-N-[4-[(4-methylpiperazin-1-yl) methyl]-3-(trifluoromethyl)phenyl]indoline-6-carboxamide (Example 17)

To a solution of Intermediate 30 (110 mg, 0.206 mmol) in water (0.5 mL) was added hydrido(dimethylphosphinous acid-kP)[hydrogen bis(dimethylphosphinito-kP)]platinum(II) (8.8 mg, 0.021 mmol) and the mixture was stirred at 100°C for 2 hours. The reaction mixture was concentrated and the residue purified by preparative HPLC (Sunfire C18 19x150mm, 10um 20-80% ACN / H₂O (10mM NH₄CO₃), 20ml/min, RT) to give the title compound (41 mg, 34%).

LC-MS (ESI): (Method 3) t*_{R}* = 3.02 min; m/z [M+H]⁺= 553.5

¹H NMR (400 MHz, DMSO) d 10.30 (s, 1H), 8.98 (d, J=2.1 Hz, 1H), 8.74 (d, J=2.0 Hz, 1H), 8.24 - 8.19 (m, 3H), 8.03 (dd, J=1.9, 8.5 Hz, 1H), 7.70 (d, J=8.5 Hz, 1H), 7.62 (s, 1H), 7.30 (dd, J=1.3, 7.6 Hz, 1H), 7.24 - 7.19 (m, 2H), 4.47 (s, 2H), 3.57 (s, 2H), 3.44 - 3.35 (m, 2H), 3.02 (dd, J=8.3, 8.3 Hz, 2H), 2.39 - 2.34 (m, 6H), 2.17 (s, 3H). 2 piperazine protons under solvent peaks.

### Comparative newly synthesised compounds lacking a linker between the indoline ring and the Hy group whereas, in that position, the compounds of the present invention show a linker -CH_{2-.}

### Example C1: Preparation of N-(3-((4-methylpiperazin-1-yl)methyl)-5-(trifluoromethyl)phenyl)-1-(pyrimidin-5-yl)indoline-6-carboxamide

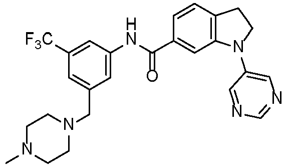

### Example C1

A 10 mL vial, equipped with a magnetic stir bar and fitted with a screw-lid, was charged with Intermediate 8 (0.030 g, 0.072 mmol) obtained as for Example 4, step 1-4, Cesium carbonate (0.070 g, 0.215 mmol), Bis(dibenzylideneacetone)palladium (4.12 mg, 7.17 µmol), 2-dicyclohexylphosphino-2',6'-diisopropoxy-1,1'-biphenyl (6.69 mg, 0.014 mmol) and 5-bromopyrimidine (0.015 g, 0.093 mmol). The vial was filled with Toluene (4 ml) and bubbled with Argon for 5 minutes. The solution was heated at 105 °C for 3h. After cooling down to room temperature, the solution was filtered through a pad of Celite, then concentrated to dryness. The crude was purified by column chromatography eluting with DCM/MeOH, from 100:0 to 90:10 to provide the title compound (11mg, 30%).

| **Example No.** | **Analytical Data** |
|---|---|
| C1 | ¹HNMR (DMSO-d6, 400MHz): d = 10.41 (s, 1H), 8.86 (s, 2H), 8.78 (s, 1H), 8.12 (s, 1H), 7.93 (s, 1H), 7.67 (s, 1H), 7.45 (d, *J*=7.7 Hz, 1H), 7.36 (br d, *J*=7.7 Hz, 1H), 7.31 (s, 1H), 4.07 (br t, *J*=8.6 Hz, 2H), 3.50 (s, 2H), 3.21 (br t, *J*=8.6 Hz, 2H), 2.26-2.42 (m, 8H), 2.12 ppm (s, 3H) |
| | LC-MS (ESI): (Method 2) t*_{R}* = 0.63 min; m/z (M+1)= 497 |

### Example C2: Preparation of N-(3-(4-methyl-1H-imidazol-1-yl)-5-(trifluoromethyl)phenyl)-1-(pyrimidin-5-yl)indoline-6-carboxamide

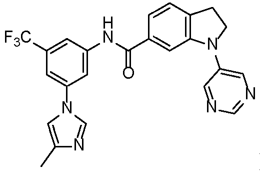

### Example C2

### Step 1; methyl indoline-6-carboxylate - Intermediate 15

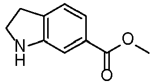

1-(tert-butyl) 6-methyl indoline-1,6-dicarboxylate (0.1 g, 0.361 mmol) was dissolved in a 3:1 mixture of DCM (6 ml) and 2,2,2-trifluoroacetic acid (2.1 ml). The solution was stirred at room temperature for 3 h. The solvents were evaporated and residue was used as it was (65 mg, 100%).

¹H NMR (DMSO-d₆, 400MHz): δ = 7.15 (d, 1H, *J =* 7.7 Hz), 7.11 (d, 1H, *J =* 7.7 Hz), 7.01 (s, 1H), 5.74 (s, 1H), 3.78 (s, 3H), 3.46 (t, 2H, *J =* 8.6 Hz), 2.96 (t, 2H, *J =* 8.6 Hz) ppm

### Step 2; methyl 1-(pyrimidin-5-yl)indoline-6-carboxylate - Intermediate 16

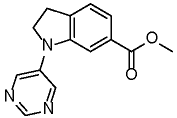

A 50mL flask, equipped with a magnetic stir bar and fitted with a rubber septum, was charged with Intermediate 15 (0.065 g, 0.367 mmol), 5-bromopyrimidine (0.076 g, 0.477 mmol), Cesium carbonate (0.359 g, 1.100 mmol), Bis(dibenzylideneacetone)palladium (0.021 g, 0.037 mmol) and 2-dicyclohexylphosphino-2',6'-diisopropoxy-1,1'-biphenyl (0.034 g, 0.073 mmol). The vessel vas evacuated and backfilled with Argon, then Toluene (5 ml) was added via syringe. The solution was heated at 100°C and stirred for 16 h. After cooling down to room temperature, the solution was filtered through a pad of Celite, then concentrated to dryness. The crude was purified by column chromatography eluting with Hept/EtOAc, from 100:0 to 0:100 to provide the title compound (87 mg, 93%),

¹H NMR (DMSO-d₆, 400MHz): δ = 8.81 (s, 3H), 7.62 (s, 1H), 7.48 (d, 1H, J = 7.5 Hz), 7.37 (d, 1H, J = 7.5 Hz), 4.09 (t, 2H, J = 8.5 Hz), 3.83 (s, 3H), 3.23 (t, 2H, J = 8.5 Hz) ppm

### Step 3; N-(3-(4-methyl-1H-imidazol-1-yl)-5-(trifluoromethyl)phenyl)-1-(pyrimidin-5-yl)indoline-6-carboxamide (Example C2)

In a 10mL vial, Intermediate 16 (0.03 g, 0.118 mmol) and 3-(4-methyl-1H-imidazol-1-yl)-5-(trifluoromethyl)aniline (0.028 g, 0.118 mmol) were dissolved in anhydrous THF (5 ml). The solution was cooled down to 0 °C (ice-bath) then potassium tert-butylate (0.040 g, 0.353 mmol) was added in one portion. The solution was stirred at room temperature for 5 h. Additional potassium tert-butylate (0.040 g, 0.353 mmol) as well as anhydrous DMF (2.0 ml) were added to the solution and stirring was maintained overnight. The reaction mixture was quenched with ice water (5 mL), then extraction with EtOAc (10 mL) was done. The organic phase was dried over Na₂SO₄, filtered and concentrated to dryness. The crude was purified by column chromatography eluting with DCM/MeOH, from 100:0 to 90:10 to provide the title compound (4.6 mg, 8%).

| **Example No.** | **Analytical Data** |
|---|---|
| C2 | ¹H NMR (DMSO-d6, 400MHz): d = 10.60 (s, 1H), 8.89 (s, 2H), 8.81 (s, 1H), 8.25-8.30 (m, 1H), 8.20 (s, 1H), 8.11 (br s, 1H), 7.67-7.75 (m, 2H), 7.46-7.54 (m,2H), 7.43 (s, 1H), 4.12 (br t, J=8.4 Hz, 2H), 3.22-3.27 (m, 2H), 2.18 ppm (s, 3H) |
| | LC-MS (ESI): (Method 2) t*_{R}* = 0.67 min; m/z (M+1)= 465 |

### Example C3: Preparation of 3-((4-methylpiperazin-1-yl)methyl)-N-(1-(pyrimidin-5-yl)indolin-6-yl)-5-(trifluoromethyl)benzamide

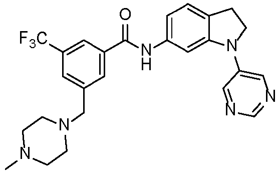

### Example C3

### Step 1; 3-((4-methylpiperazin-1-yl)methyl)-N-(1-(pyrimidin-5-yl)indolin-6-yl)-5-(trifluoromethyl)benzamide

A 40mL vial, equipped with a magnetic stir bar and fitted with a rubber septum, was charged with Intermediate 12 (0.095 g, 0.227 mmol), 5-bromopyrimidine (0.047 g, 0.295 mmol), Cesium carbonate (0.222 g, 0.681 mmol), Bis(dibenzylideneacetone)palladium (0.013 g, 0.023 mmol) and 2-dicyclohexylphosphino-2',6'-diisopropoxy-1,1'-biphenyl (0.021 g, 0.045 mmol) and dissolved in Toluene (5 ml). The vessel was bubbled with Argon for 10min, then the solution was heated at 100 °C for 16h. After cooling down to room temperature, the solution was filtered through a pad of Celite, then concentrated to dryness. The crude was purified on FCC eluting with H₂O/MeCN/HCO₂H, from 95:5:0.1 to 5:95:0.1 to provide the title compound as formate salt (123 mg, 100 %).

| **Example No.** | **Analytical Data** |
|---|---|
| C3 | ¹H NMR (DMSO-d6, 400MHz): d = 10.35 (s, 1H), 8.79 (s, 2H), 8.77 (s, 1H), 8.12-8.20 (m, 3H), 7.85 (s, 1H), 7.74 (d, *J=*1.3 Hz, 1H), 7.38 (dd, *J*=7.9, 1.5 Hz, 1H), 7.23 (d, *J*=7.9 Hz, 1H), 4.06 (t, *J*=8.4 Hz, 2H), 3.65 (s, 2H), 3.14 (br t, *J=*8.4 Hz, 2H), 2.29-2.49 (m, 8H), 2.20 ppm (s, 3H) LC-MS (ESI): (Method 2) t*_{R}=* 0.61 min; m/z (M+1)= 497 |

### PHARMACOLOGICAL ACTIVITY OF THE COMPOUNDS OF THE INVENTION

### In vitro Assays

### Binding Assays

DDR1 and DDR2 binding assays were performed using Life Technologies LanthaScreen^{™} Europium Kinase Binding assay. The compounds were incubated with 5 nM DDR1 (Carna Biosciences) or 5 nM DDR2 (Life Technologies) for 1 hour at room temperature in white 384-well OptiPlate (PerkinElmer), containing 20 nM or 10 nM Kinase Tracer 178 respectively and 2 nM Europium labelled anti-GST antibody (Life Technologies) in assay buffer (50 mM HEPES pH 7.5, 10 mM MgCI2, 1 mM EGTA and 0.01% BRIJ35). The ratio of fluorescence emission 665 nm/ 615 nm after excitation at 340 nm was obtained using the Tecan Spark 20M plate reader. IC50 values were determined in GraphPad Prism 8.0 software, using 4 parameter model: log(inhibitor) vs. response. IC50 values were converted in Ki using the Cheng-Prusoff equation (Ki=IC50/(1+[Tracer]/Kd).

### DDR1 cell based assay

The inhibition of DDR1 receptor activation by compounds was evaluated by PathHunter^{®} U2OS DDR1 assay (Eurofins DiscoverX), according to the manufacturer's instructions. Briefly, U2OS-DDR1 cells were seeded in white 384-well plates at a density of 5000 cells/well and incubated for 2 hours at 37°C and 5% CO₂. Cells were then treated with compounds at different concentrations and incubated for 30 minutes, before stimulation with bovine Type II Collagen 20 µg/ml and incubation overnight at 37°C and 5% CO₂. PathHunter Detection Reagents were prepared according to the protocol provided by DiscoverX and 20 µl/well of this mix were added to each well. After incubating the plates for 1 hour at room temperature in the dark, luminescence signal was acquired with a plate reader. Raw data were normalized to vehicle control (0% for normalization) and positive control (100% for normalization; cells treated with 20 µg/ml collagen II) and IC50 parameters were calculated in GraphPad Prism 8.0 software, using sigmoidal dose-response curve fitting with variable slope.

### DDR2 cell based assay

The inhibition of DDR2 phosphorylation by compounds was evaluated in HEK293T-DDR2 recombinant cells by phospho-ELISA assay. Briefly, HEK293T-DDR2 cells were seeded in poly-D-lysine-coated 24-well plates at a density of 250.000 cells/well and incubated for 1.5 hours at 37°C and 5% CO₂ in DMEM + 10% FBS. After that, the medium was changed to serum-free DMEM and cells were incubated for 3 hours. Then. test compounds were added at different concentrations 30 minutes before stimulation with bovine Type II Collagen at 50 µg/ml for further 3 hours. For DDR2 phospho-ELISA assay (DuoSet IC Human Phospho-DDR2; R&D Systems), protein extracts were obtained by adding 60 µl/well of lysis buffer prepared according to the manufacturer's instructions. Protein concentration in the samples was determined by BCA assay and the levels of phospho-DDR2 were determined following R&D Systems indications. Raw data were normalized to maximal inhibition control (0% for normalization) and positive control (100% for normalization; cells treated with 20 µg/ml collagen II) and IC50 parameters were calculated in GraphPad Prism 8.0 software, using sigmoidal dose-response curve fitting with variable slope.

The results for individual compounds are provided below in Table 5 wherein the compounds are classified in term of binding affinity for DDR1 and DDR2 (Ki) and in term of potency (IC50) with respect to their inhibitory activity on DDR1 and DDR2 receptors.

**Table 5**

| **Example No.** | **Ki DDR1** | **Ki DDR2** | **IC50 DDR1** | **IC50DDR2** |
|---|---|---|---|---|
| 1 | +++ | +++ | + | - |
| 2 | +++ | +++ | ++ | - |
| 3 | +++ | +++ | + | - |
| 4 | +++ | +++ | +++ | +++ |
| 5 | +++ | +++ | +++ | ++ |
| 6 | +++ | +++ | ++ | - |
| 7 | +++ | +++ | +++ | - |
| 8 | +++ | ++ | +++ | - |
| 9 | +++ | ++ | +++ | - |
| 10 | +++ | +++ | +++ | - |
| 11 | +++ | +++ | +++ | - |
| 12 | +++ | ++ | +++ | - |
| 13 | +++ | ++ | +++ | + |
| 14 | +++ | ++ | +++ | + |
| 15 | +++ | - | +++ | - |
| 16 | +++ | +++ | +++ | +++ |
| 17 | +++ | +++ | ++ | - |
| 18 | +++ | +++ | +++ | +++ |
| 19 | +++ | +++ | +++ | - |
| 20 | +++ | +++ | + | - |
| 21 | +++ | +++ | + | - |

| | | | | |
|---|---|---|---|---|
| +: Ki between 50 and 80 nM ++: Ki between 25 and 50 nM +++: Ki lower than 25 nM +: IC50 between 50 and 80 nM ++: IC50 between 25 and 50 nM +++: IC50 lower than 25 nM : not available | | | | |

As it can be appreciated, the compounds of Table 5, i.e. the compounds of the invention, show a good activity as antagonists of DDR1 and DDR2 receptors. Accordingly, the compounds of the invention can be effectively used for treating disease, disorder or condition associated with DDR receptors, such as fibrosis, e.g. pulmonary fibrosis, idiopathic pulmonary fibrosis (IPF), hepatic fibrosis, renal fibrosis, ocular fibrosis, cardiac fibrosis, arterial fibrosis and systemic sclerosis.

### Comparative Examples

Compounds of the Examples C1 to C3 were tested in the same binding assay described above.

**Table 6**

| **Example No** | **DDR1 Ki (nM)** | **DDR2 Ki (nM)** |
|---|---|---|
| C1 | 30000 | 50000 |
| C2 | 58000 | 4000 |
| C3 | 58000 | 58000 |

The compounds of the present invention, as shown in Table 5, have both a binding affinity for DDR1 and DDR2 receptors expressed as Ki and an inhibitory potency expressed as IC50 against DDR1 and DDR2 receptors lower than 80 nM, and for most of the compounds lower than 50 nM or even lower than 25 nM. Conversely, the comparative Examples C1 to C3, as shown in Table 6, have a binding affinity (Ki) of 30000 nM or higher on DDR1 receptor, even of 58000 for C2 and C3, and higher than 4000 nM on DDR2 receptor, even equal or higher than 50000 for C1 and C3, respectively.

These data demonstrate that, conversely to the compounds of Examples C1 to C3, characterized by lacking a linker between the indoline ring and the Hy group, the presence of a -CH₂- linker in that position in the present invention compounds unexpectedly and remarkably determines a relevant increase in the inhibitory activity on the DDR1 and DDR2 receptors.

## Claims

1. A compound of formula (I) 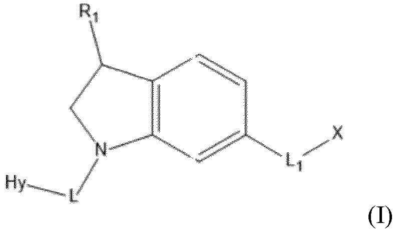 wherein
**R₁** is H or -(C₁-C₄)alkyl;
**L** is -CH₂;
**L₁** is selected from the group consisting of -C(O)NH- and -NHC(O)-;
**Hy** is a monocyclic heteroaryl optionally substituted by one or more groups selected from -(CH₂)ₙNR₄R₅, -C(O)NR₄R₅, -(C₁-C₄)alkyl, -NR₄(CO)R₅, -(C₁-C₄)alkylene-O-(C₁-C₄)alkyl and -(C₄-C₇)heterocycloalkyl optionally substituted by one or more groups selected from -(C₁-C₄)alkyl and oxo;
n is 0, 1 or 2;
**R₄** is H or -(C₁-C₄)alkyl;
**R₅** is H or is selected from the group consisting of (C₃-C₇)cycloalkyl, -(C₁-C₄)alkyl and heteroaryl optionally substituted by one or more groups selected from -(C₁-C₄)alkyl,
-(C₁-C₄)alkylene-NR₁R₄ and -(C₁-C₄)alkylene-O-(C₁-C₄)alkyl;
**X** is 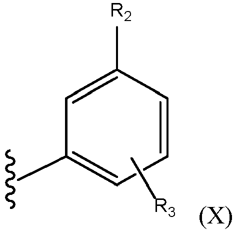
wherein **R₂** is selected from the group consisting of -(C₁-C₄)alkyl, -O(C₁-C₄)haloalkyl, halogen atoms, -(C₁-C₄)haloalkyl, -(C₃-C₇)cycloalkyl and -O(C₃-C₇)cycloalkyl;
**R₃** is H or is selected from the group consisting of -O(C₁-C₄)alkyl, heteroaryl optionally substituted by one or more -(C₁-C₄)alkyl, and (C₄-C₇)heterocycloalkyl-(C₁-C₄)alkylene-, wherein said -(C₄-C₇)heterocycloalkyl is optionally substituted by one or more groups selected from -(C₁-C₄)alkyl and -O(C₁-C₄)alkyl;
and pharmaceutically acceptable salts thereof.

2. The compound of formula (I) according to claim 1, wherein **L₁** is -C(O)NH- and **X** is X' 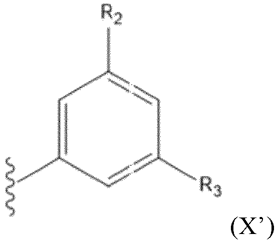 represented by the formula (Ia1')

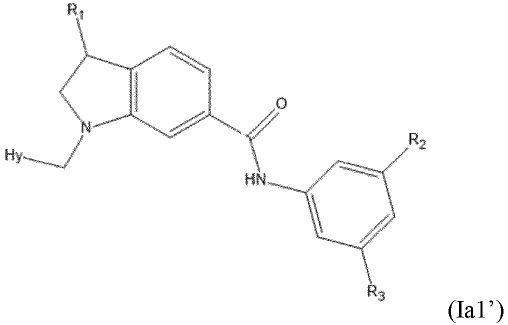

3. The compound of formula (Ia1') according to claim 2 selected from at least one of:
N-(3-(4-methyl-1H-imidazol-1-yl)-5-(trifluoromethyl)phenyl)-1-(pyrimidin-5-ylmethyl)indoline-6-carboxamide;
N-(3-(4-methyl-1H-imidazol-1-yl)-5-(trifluoromethyl)phenyl)-1-((2-(methylcarbamoyl)pyridin-4-yl)methyl)indoline-6-carboxamide;
N-(3-((4-methylpiperazin-1-yl)methyl)-5-(trifluoromethyl)phenyl)-1-(pyrimidin-5-ylmethyl)indoline-6-carboxamide;
1-((2-((1-methyl-1H-pyrazol-4-yl)amino)pyrimidin-5-yl)methyl)-N-(3-((4-methylpiperazin-1-yl)methyl)-5-(trifluoromethyl)phenyl)indoline-6-carboxamide;
1-((2-((1-(2-methoxyethyl)-1H-pyrazol-4-yl)amino)pyrimidin-5-yl)methyl)-N-(3-((4-methylpiperazin-1-yl)methyl)-5-(trifluoromethyl)phenyl)indoline-6-carboxamide; and
1-((2-((1-(2-(dimethylamino)ethyl)-1H-pyrazol-4-yl)amino)pyrimidin-5- yl)methyl)-N-(3-((4-methylpiperazin-1-yl)methyl)-5-
(trifluoromethyl)phenyl)indoline-6-carboxamide.

4. The compound of formula (I) according to claim 1, wherein L₁ is -C(O)NH- and X is X'' 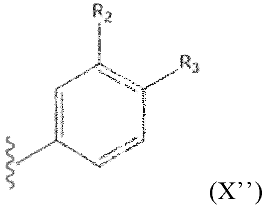 represented by the formula (Ia1'')

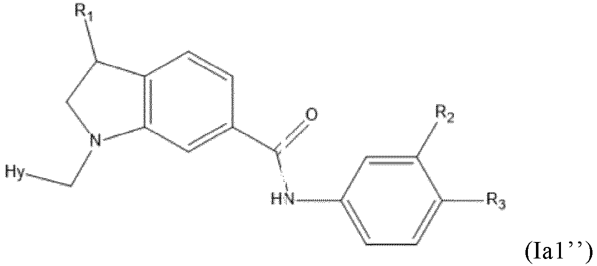

5. The compound of formula (Ia1") according to claim 4, selected from at least one of:
1-((2-aminopyrimidin-5-yl)methyl)-N-(4-((4-methylpiperazin-1-yl)methyl)-3-(trifluoromethyl)phenyl)indoline-6-carboxamide;
1-((6-aminopyridin-3-yl)methyl)-N-(4-((4-methylpiperazin-1-yl)methyl)-3-(trifluoromethyl)phenyl)indoline-6-carboxamide;
1-((4-aminopyrimidin-5-yl)methyl)-N-(4-((4-methylpiperazin-1-yl)methyl)-3-(trifluoromethyl)phenyl)indoline-6-carboxamide;
1-((5-aminopyrazin-2-yl)methyl)-N-(4-((4-methylpiperazin-1-yl)methyl)-3-(trifluoromethyl)phenyl)indoline-6-carboxamide;
1-((2-(methylamino)pyrimidin-5-yl)methyl)-N-(4-((4-methylpiperazin-1-yl)methyl)-3-(trifluoromethyl)phenyl)indoline-6-carboxamide;
1-((6-acetamidopyridin-3-yl)methyl)-N-(4-((4-methylpiperazin-1-yl )methyl)-3-(trifluoromethyl)phenyl)indoline-6-carboxamide;
1-((6-(methylamino)pyridin-3-yl)methyl)-N-(4-((4-methylpiperazin-1-yl)methyl)-3-(trifluoromethyl)phenyl)indoline-6-carboxamide;
1-((2-acetamidopyrimidin-5-yl)methyl)-N-(4-((4-methylpiperazin-1-yl)methyl)-3-(trifluoromethyl)phenyl)indoline-6-carboxamide; and
1-((5-carbamoylpyridin-3-yl)methyl)-N-(4-((4-methylpiperazin-1-yl)methyl)-3-(trifluoromethyl)phenyl)indoline-6-carboxamide.

6. The compound of formula (I) according to claim 1, wherein **Li** is -NHC(O)- and X is X' 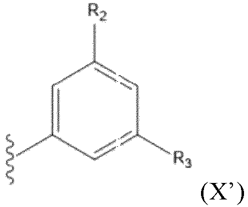 represented by the formula (Ib1')

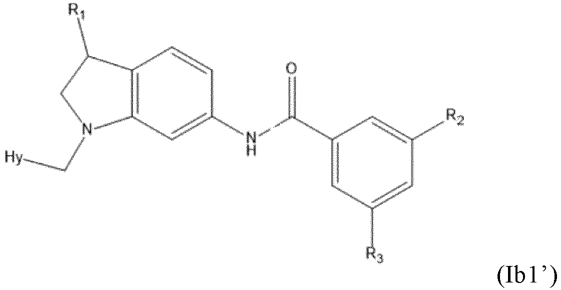

7. The compound of formula (Ib1') according to claim 6, selected from at least one of:
N-(1-((2-((1-methyl-1H-pyrazol-4-yl)amino)pyrimidin-5-yl)methyl)indolin-6-yl)-3-((4-methylpiperazin-1-yl)methyl)-5-(trifluoromethyl)benzamide;
N-(1-((3-aminopyrazin-2-yl)methyl)indolin-6-yl)-3-((4-methylpiperazin-1-yl)methyl)-5-(trifluoromethyl)benzamide;
N-methyl-4-((6-(3-(4-methyl-1H-imidazol-1-yl)-5-(trifluoromethyl)benzamido)indolin-1-yl)methyl)picolinamide; and
N-(1-((2-aminopyrimidin-5-yl)methyl)indolin-6-yl)-3-((4-methylpiperazin-1-yl)methyl)-5-(trifluoromethyl)benzamide.

8. A pharmaceutical composition comprising a compound of formula (I) according to any one of claims 1 to 7, in admixture with one or more pharmaceutically acceptable carrier or excipient.

9. The pharmaceutical composition according to claim 8 for administration by inhalation.

10. The compound of formula (I) according to any one of claims 1 to 7 or the pharmaceutical composition according to claim 8 or 9 for use as a medicament.

11. The compound of formula (I) or the pharmaceutical composition for use according to claim 10 in the prevention and/or treatment of a disease, disorder or condition associated with dysregulation of Discoidin Domain Receptors.

12. The compound of formula (I) or the pharmaceutical composition for use according to claim 10 or 11 in the prevention and/or treatment of fibrosis and/or diseases, disorders or conditions that involve fibrosis.

13. The compound of formula (I) or the pharmaceutical composition for use according to claim 12 in the prevention and/or treatment of fibrosis including pulmonary fibrosis, idiopathic pulmonary fibrosis (IPF), hepatic fibrosis, renal fibrosis, ocular fibrosis, cardiac fibrosis, arterial fibrosis and systemic sclerosis.

14. The compound of formula (I) or the pharmaceutical composition for use according to claim 13 in the prevention and/or treatment of idiopathic pulmonary fibrosis (IPF).

## Patentansprüche

1. Verbindung der Formel (I), 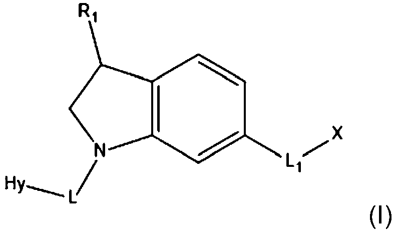 wobei
R₁ H oder -(C₁-C₄)Alkyl ist;
L -CH₂ ist;
L₁ aus der Gruppe ausgewählt ist, bestehend aus -C(O)NH- und - NHC(O)-;
**Hy** ein monozyklisches Heteroaryl ist, das optional durch eine oder mehrere Gruppen substituiert ist, die ausgewählt sind aus -(CH₂)ₙNR₄R₅, -C(O)NR₄R₅, -(C₁-C₄)Alkyl, -NR₄(CO)R₅, -(C₁-C₄)Alkylen-O-(C₁-C₄)Alkyl und -(C₄-C₇)Heterocycloalkyl, das optional durch eine oder mehrere Gruppen substituiert ist, die ausgewählt sind aus -(C₁-C₄)Alkyl und Oxo;
**n** 0, 1 oder 2 ist;
**R₄** H oder -(C₁-C₄)Alkyl ist;
**R₅** H ist oder aus der Gruppe ausgewählt ist, bestehend aus (C₃-C₇)Cycloalkyl, -(C₁-C₄)Alkyl und Heteroaryl, optional substituiert durch eine oder mehrere Gruppen, die ausgewählt sind aus -(C₁-C₄)Alkyl,
-(C₁-C₄)Alkylen-NR₁R₄ und -(C₁-C₄)Alkylen-O-(C₁-C₄)Alkyl;
**X** ist 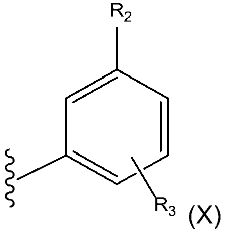
wobei **R₂** aus der Gruppe ausgewählt ist, bestehend aus -(C₁-C₄)Alkyl, - O(C₁-C₄)Haloalkyl, Halogenatomen, -(C₁-C₄)Haloalkyl, -(C₃-C₇)Cycloalkyl und -O(C₃-C₇)Cycloalkyl;
**R₃** H ist oder aus der Gruppe ausgewählt ist, bestehend aus -O(C₁-C₄)Alkyl, Heteroaryl, das optional durch ein oder mehrere -(C₁-C₄)Alkyle substituiert ist, und (C₄-C₇)Heterocycloalkyl-(C₁-C₄)Alkylen-, wobei das -(C₄-C₇)Heterocycloalkyl optional durch eine oder mehrere Gruppen substituiert ist, die ausgewählt sind aus -(C₁-C₄)Alkyl und -O(C₁-C₄)Alkyl;
und pharmazeutisch annehmbare Salze davon.

2. Verbindung der Formel (I) nach Anspruch 1, wobei **L₁** -C(O)NH- ist und **X** X' ist, 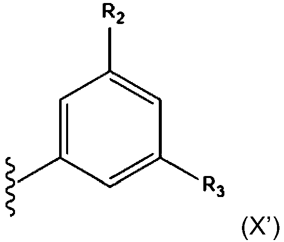 dargestellt durch die Formel (la1')

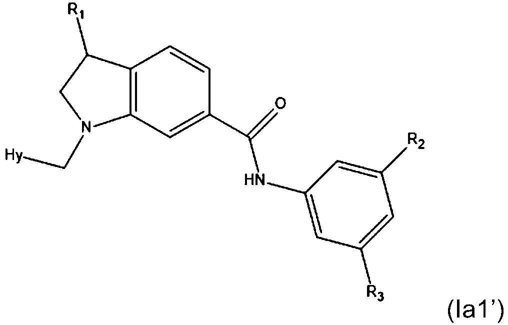

3. Verbindung der Formel (la1') nach Anspruch 2, die ausgewählt ist aus mindestens einem von:
N-(3-(4-Methyl-1H-imidazol-1-yl)-5-(trifluormethyl)phenyl)-1-(pyrimidin-5-ylmethyl)indolin-6-carboxamid;
N-(3-(4-Methyl-1H-imidazol-1-yl)-5-(trifluormethyl)phenyl)-1-((2-(methylcarbamoyl)pyridin-4-yl)methyl)indolin-6-carboxamid;
N-(3-((4-Methylpiperazin-1-yl)methyl)-5-(trifluormethyl)phenyl)-1-(pyrimidin-5-ylmethyl)indolin-6-carboxamid;
1-((2-((1-Methyl-1H-pyrazol-4-yl)amino)pyrimidin-5-yl)methyl)-N-(3-((4-methylpiperazin-1-yl)methyl)-5-(trifluormethyl)phenyl)indolin-6-carboxamid;
1-((2-((1-(2-Methoxyethyl)-1H-pyrazol-4-yl)amino)pyrimidin-5-yl)methyl)-N-(3-((4-methylpiperazin-1-yl)methyl)-5-(trifluormethyl)phenyl)indolin-6-carboxamid; und
1-((2-((1-(2-(Dimethylamino)ethyl)-1H-pyrazol-4-yl)amino)pyrimidin-5-yl)methyl)-N-(3-((4-methylpiperazin-1-yl)methyl)-5-(trifluormethyl)phenyl)indolin-6-carboxamid.

4. Verbindung der Formel (I) nach Anspruch 1, wobei L₁ -C(O)NH- ist und **X** X" ist, 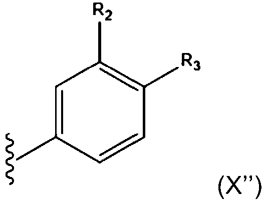 dargestellt durch die Formel (la1")

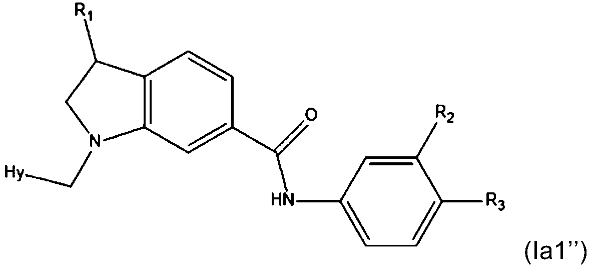

5. Verbindung der Formel (la1") nach Anspruch 4, die ausgewählt ist aus mindestens einem von:
1-((2-Aminopyrimidin-5-yl)methyl)-N-(4-((4-methylpiperazin-1-yl)methyl)-3-(trifluormethyl)phenyl)indolin-6-carboxamid;
1-((6-Aminopyridin-3-yl)methyl)-N-(4-((4-methylpiperazin-1-yl)methyl)-3-(trifluormethyl)phenyl)indolin-6-carboxamid;
1-((4-Aminopyrimidin-5-yl)methyl)-N-(4-((4-methylpiperazin-1-yl)methyl)-3-(trifluormethyl)phenyl)indolin-6-carboxamid;
1-((5-Aminopyrazin-2-yl)methyl)-N-(4-((4-methylpiperazin-1-yl)methyl)-3-(trifluormethyl)phenyl)indolin-6-carboxamid;
1-((2-(Methylamino)pyrimidin-5-yl)methyl)-N-(4-((4-methylpiperazin-1-yl)methyl)-3-(trifluormethyl)phenyl)indolin-6-carboxamid;
1-((6-Acetamidopyridin-3-yl)}methyl)-N-(4-((4-methylpiperazin-1-yl)methyl)-3-(trifluormethyl)phenyl)indolin-6-carboxamid;
1-((6-(Methylamino)pyridin-3-yl)methyl)-N-(4-((4-methylpiperazin-1-yl)methyl)-3-(trifluormethyl)phenyl)indolin-6-carboxamid;
1-((2-Acetamidopyrimidin-5-yl)methyl)-N-(4-((4-methylpiperazin-1-yl)methyl)-3-(trifluormethyl)phenyl)indolin-6-carboxamid; und
1-((5-Carbamoylpyridin-3-yl)methyl)-N-(4-((4-methylpiperazin-1-yl)methyl)-3-(trifluormethyl)phenyl)indolin-6-carboxamid.

6. Verbindung der Formel (I) nach Anspruch 1, wobei L₁ -NHC(O)- ist und X X' ist, 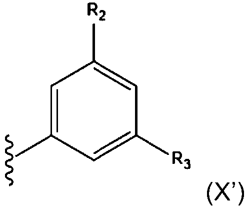 dargestellt durch die Formel (Ib1')

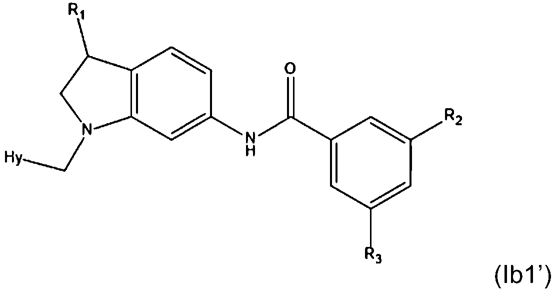

7. Verbindung der Formel (Ib1') nach Anspruch 6, die ausgewählt ist aus mindestens einem von:
N-(1-((2-((1-Methyl-1H-pyrazol-4-yl)amino)pyrimidin-5-yl)methyl)indolin-6-yl)-3-((4-methylpiperazin-1-yl)methyl)-5-(trifluormethyl)benzamid;
N-(1-((3-Aminopyrazin-2-yl)methyl)indolin-6-yl)-3-((4-methylpiperazin-1-yl)methyl)-5-(trifluormethyl)benzamid;
N-Methyl-4-((6-(3-(4-methyl-1H-imidazol-1-yl)-5-(trifluormethyl)benzamido)indolin-1-yl)methyl)picolinamid; und
N-(1-((2-Aminopyrimidin-5-yl)methyl)indolin-6-yl)-3-((4-methylpiperazin-1-yl)methyl)-5-(trifluormethyl)benzamid.

8. Pharmazeutische Zusammensetzung, umfassend eine Verbindung der Formel (I) nach einem der Ansprüche 1 bis 7 unter Beigabe eines oder mehrerer pharmazeutisch annehmbarer Träger oder Hilfsstoffe.

9. Pharmazeutische Zusammensetzung nach Anspruch 8 zur Verabreichung durch Inhalation.

10. Verbindung der Formel (I) nach einem der Ansprüche 1 bis 7 oder pharmazeutische Zusammensetzung nach Anspruch 8 oder 9 zur Verwendung als ein Arzneimittel.

11. Verbindung der Formel (I) oder pharmazeutische Zusammensetzung zur Verwendung nach Anspruch 10 bei der Prävention und/oder Behandlung einer Erkrankung, einer Störung oder eines Zustands, die/der mit einer Dysregulation von Discoidin-Domänenrezeptoren assoziiert ist.

12. Verbindung der Formel (I) oder pharmazeutische Zusammensetzung zur Verwendung nach Anspruch 10 oder 11 bei der Prävention und/oder Behandlung von Fibrose und/oder Erkrankungen, Störungen oder Zuständen, die mit Fibrose einhergehen.

13. Verbindung der Formel (I) oder pharmazeutische Zusammensetzung zur Verwendung nach Anspruch 12 bei der Prävention und/oder Behandlung von Fibrose, einschließlich pulmonaler Fibrose, idiopathischer pulmonaler Fibrose (IPF), hepatischer Fibrose, renaler Fibrose, okularer Fibrose, kardialer Fibrose, arterieller Fibrose und systemischer Sklerose.

14. Verbindung der Formel (I) oder pharmazeutische Zusammensetzung zur Verwendung nach Anspruch 13 bei der Prävention und/oder Behandlung von idiopathischer pulmonaler Fibrose (IPF).

## Revendications

1. Composé de formule (I) 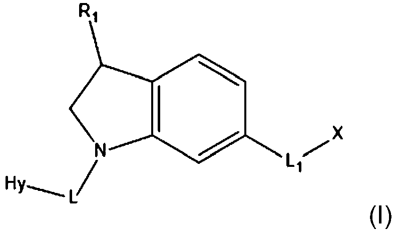 dans lequel
**R₁** est H ou -(C₁-C₄)alkyle ;
**L** est -CH₂ ;
**L₁** est choisi dans le groupe constitué de -C(O)NH- et -NHC(O)- ;
**Hy** est un hétéroaryle monocyclique éventuellement substitué par un ou plusieurs groupes choisis parmi -(CH₂)ₙNR₄R₅, -C(O)NR₄R₅, -(C₁-C₄)alkyle, - NR₄(CO)R₅, -(C₁-C₄)alkylène-O-(C₁-C₄)alkyle et -(C₄-C₇)hétérocycloalkyle éventuellement substitué par un ou plusieurs groupes choisis parmi -(C₁-C₄)alkyle et oxo ;
**n** vaut 0, 1 ou 2 ;
**R₄** est H ou -(C₁-C₄)alkyle ;
**R₅** est H ou est choisi dans le groupe constitué de (C₃-C₇)cycloalkyle, -(C₁-C₄)alkyle et hétéroaryle éventuellement substitués par un ou plusieurs groupes choisis parmi -(C₁-C₄)alkyle,
-(C₁-C₄)alkylène-NR₁R₄ et -(C₁-C₄)alkylène-O-(C₁-C₄)alkyle ;
X est 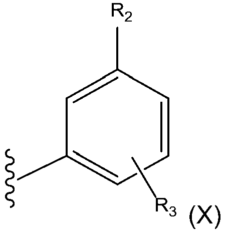
dans lequel **R₂** est choisi dans le groupe constitué de -(C₁-C₄)alkyle, -O(C₁-C₄)haloalkyle, atomes d'halogène, -(C₁-C₄)haloalkyle, -(C₃-C₇)cycloalkyle et -O(C₃-C₇)cycloalkyle ;
**R₃** est H ou est choisi dans le groupe constitué de -O(C₁-C₄)alkyle, hétéroaryle éventuellement substitué par un ou plusieurs parmi -(C₁-C₄)alkyle, et (C₄-C₇)hétérocycloalkyl-(C₁-C₄)alkylène-, dans lequel ledit -(C₄-C₇)hétérocycloalkyle est éventuellement substitué par un ou plusieurs groupes choisis parmi -(C₁-C₄)alkyle et -O(C₁-C₄)alkyle ;
et ses sels acceptables du point de vue pharmaceutique.

2. Composé de formule (I) selon la revendication 1, dans lequel **L₁** est -C(O)NH- et **X** est X' 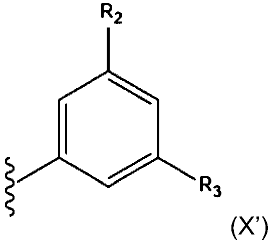 représenté par la formule (la1')

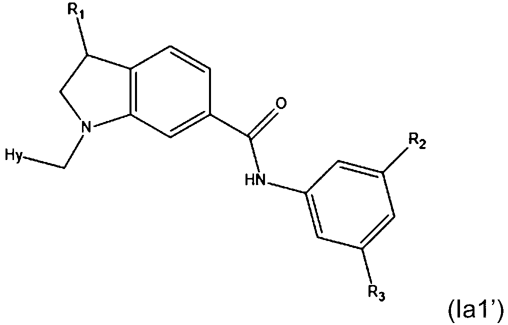

3. Composé de formule (la1') selon la revendication 2 choisi parmi au moins l'un des composés suivants :
N-(3-(4-méthyl-1H-imidazol-1-yl)-5-(trifluorométhyl)phényl)-1-(pyrimidin-5-ylméthyl)indoline-6-carboxamide ;
N-(3-(4-méthyl-1H-imidazol-1-yl)-5-(trifluorométhyl)phényl)-1-((2-(méthylcarbamoyl)pyridin-4-yl)méthyl)indoline-6-carboxamide ;
N-(3-((4-méthylpipérazin-1-yl)méthyl)-5-(trifluorométhyl)phényl)-1-(pyrimidin-5-ylméthyl)indoline-6-carboxamide ;
1-((2-((1-méthyl-1H-pyrazol-4-yl)amino)pyrimidin-5-yl)méthyl)-N-(3-((4-méthylpipérazin-1-yl)méthyl)-5-(trifluorométhyl)phényl)indoline-6-carboxamide ;
1-((2-((1-(2-méthoxyéthyl)-1H-pyrazol-4-yl)amino)pyrimidin-5-yl)méthyl)-N-(3-((4-méthylpipérazin-1-yl)méthyl)-5-(trifluorométhyl)phényl)indoline-6-carboxamide ; et
1-((2-((1-(2-(diméthylamino)éthyl)-1H-pyrazol-4-yl)amino)pyrimidin-5-yl)méthyl)-N-(3-((4-méthylpipérazin-1-yl)méthyl)-5-(trifluorométhyl)phényl)indoline-6-carboxamide.

4. Composé de formule (I) selon la revendication 1, dans lequel **L₁** est -C(O)NH- et **X** est X" 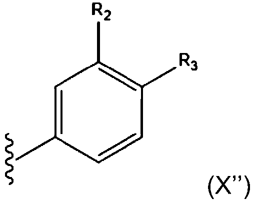 représenté par la formule (la1")

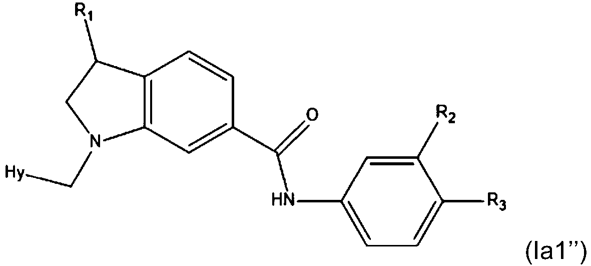

5. Composé de formule (la1") selon la revendication 4, choisi parmi au moins l'un des composés suivants :
1-((2-aminopyrimidin-5-yl)méthyl)-N-(4-((4-méthylpipérazin-1-yl)méthyl)-3-(trifluorométhyl)phényl)indoline-6-carboxamide ;
1-((6-aminopyridin-3-yl)méthyl)-N-(4-((4-méthylpipérazin-1-yl)méthyl)-3-(trifluorométhyl)phényl)indoline-6-carboxamide ;
1-((4-aminopyrimidin-5-yl)méthyl)-N-(4-((4-méthylpipérazin-1-yl)méthyl)-3-(trifluorométhyl)phényl)indoline-6-carboxamide ;
1-((5-aminopyrazin-2-yl)méthyl)-N-(4-((4-méthylpipérazin-1-yl)méthyl)-3-(trifluorométhyl)phényl)indoline-6-carboxamide ;
1-((2-(méthylamino)pyrimidin-5-yl)méthyl)-N-(4-((4-méthylpipérazin-1-yl)méthyl)-3-(trifluorométhyl)phényl)indoline-6-carboxamide ;
1-((6-acétamidopyridin-3-yl)méthyl)-N-(4-((4-méthylpipérazin-1-yl)méthyl)-3-(trifluorométhyl)phényl)indoline-6-carboxamide ;
1-((6-(méthylamino)pyridin-3-yl)méthyl)-N-(4-((4-méthylpipérazin-1-yl)méthyl)-3-(trifluorométhyl)phényl)indoline-6-carboxamide ;
1-((2-acétamidopyrimidin-5-yl)méthyl)-N-(4-((4-méthylpipérazin-1-yl)méthyl)-3-(trifluorométhyl)phényl)indoline-6-carboxamide ; et
1-((5-carbamoylpyridin-3-yl)méthyl)-N-(4-((4-méthylpipérazin-1-yl)méthyl)-3-(trifluorométhyl)phényl)indoline-6-carboxamide.

6. Composé de formule (I) selon la revendication 1, dans lequel L₁ est -NHC(O)- et X est X' 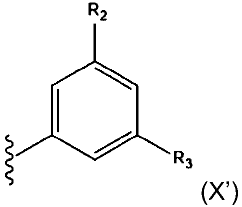 représenté par la formule (Ib1')

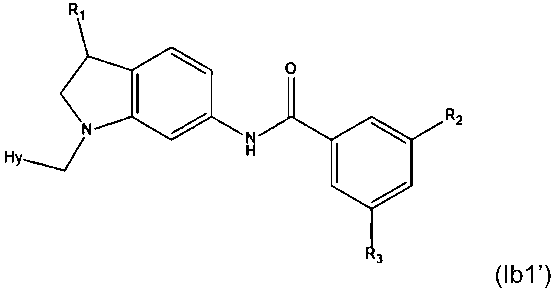

7. Composé de formule (Ib1') selon la revendication 6, choisi parmi au moins l'un des composés suivants :
N-(1-((2-((1-méthyl-1H-pyrazol-4-yl)amino)pyrimidin-5-yl)méthyl)indolin-6-yl)-3-((4-méthylpipérazin-1-yl)méthyl)-5-(trifluorométhyl)benzamide ;
N-(1-((3-aminopyrazin-2-yl)méthyl)indolin-6-yl)-3-((4-méthylpipérazin-1-yl)méthyl)-5-(trifluorométhyl)benzamide ;
N-méthyl-4-((6-(3-(4-méthyl-1H-imidazol-1-yl)-5-(trifluorométhyl)benzamido)indolin-1-yl)méthyl)picolinamide ; et
N-(1-((2-aminopyrimidin-5-yl)méthyl)indolin-6-yl)-3-((4-méthylpipérazin-1-yl)méthyl)-5-(trifluorométhyl)benzamide.

8. Composition pharmaceutique comprenant un composé de formule (I) selon l'une quelconque des revendications 1 à 7, en mélange avec un ou plusieurs transporteurs ou excipients acceptables du point de vue pharmaceutique.

9. Composition pharmaceutique selon la revendication 8 pour administration par inhalation.

10. Composé de formule (I) selon l'une quelconque des revendications 1 à 7 ou composition pharmaceutique selon la revendication 8 ou 9 pour une utilisation en tant que médicament.

11. Composé de formule (I) ou composition pharmaceutique pour une utilisation selon la revendication 10 dans la prévention et/ou le traitement d'une maladie, d'un trouble ou d'une affection associé à un dérèglement de récepteurs du domaine de la discoïdine.

12. Composé de formule (I) ou composition pharmaceutique pour une utilisation selon la revendication 10 ou 11 dans la prévention et/ou le traitement de la fibrose et/ou de maladies, troubles ou affections qui impliquent la fibrose.

13. Composé de formule (I) ou composition pharmaceutique pour une utilisation selon la revendication 12 dans la prévention et/ou le traitement de la fibrose, y compris la fibrose pulmonaire, la fibrose pulmonaire idiopathique (FPI), la fibrose hépatique, la fibrose rénale, la fibrose oculaire, la fibrose cardiaque, la fibrose artérielle et la sclérose systémique.

14. Composé de formule (I) ou composition pharmaceutique pour une utilisation selon la revendication 13 dans la prévention et/ou le traitement de la fibrose pulmonaire idiopathique (FPI).
